(19) Europäisches Patentamt
European Patent Office
Office européen des brevets

(11) EP 4 594 391 B1

(12) # EUROPÄISCHE PATENTSCHRIFT

(45) Veröffentlichungstag und Bekanntmachung des Hinweises auf die Patenterteilung:
**22.10.2025 Patentblatt 2025/43**

(21) Anmeldenummer: **23710318.9**

(22) Anmeldetag: **08.03.2023**

(51) Internationale Patentklassifikation (IPC):
***C08G 63/08*** *(2006.01)* ***C08G 63/78*** *(2006.01)*
***C08G 63/91*** *(2006.01)* ***C08G 63/664*** *(2006.01)*
***C08G 63/682*** *(2006.01)* ***C08G 63/685*** *(2006.01)*
***C08G 63/82*** *(2006.01)*

(52) Gemeinsame Patentklassifikation (CPC):
**C08G 63/08; C08G 63/664; C08G 63/6822; C08G 63/6852; C08G 63/78; C08G 63/823; C08G 63/912**

(86) Internationale Anmeldenummer:
**PCT/EP2023/055935**

(87) Internationale Veröffentlichungsnummer:
**WO 2024/183907 (12.09.2024 Gazette 2024/37)**

(54) **DERIVATE VON POLY-3-HYDROXY-ALKANOATEN UND VERFAHREN ZU DEREN HERSTELLUNG**

DERIVATIVES OF POLY-3-HYDROXY ALKANOATES AND METHOD FOR THE PREPARATION THEREOF

DÉRIVÉS DE POLY-3-HYDROXY-ALCANOATES ET LEUR PROCÉDÉ DE PRÉPARATION

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(43) Veröffentlichungstag der Anmeldung:
**06.08.2025 Patentblatt 2025/32**

(73) Patentinhaber: **Wacker Chemie AG**
**81671 München (DE)**

(72) Erfinder: **STEPP, Michael**
**81671 Munich (DE)**

(56) Entgegenhaltungen:
**CN-A- 109 776 312**

- **MICHALAK MICHAL ET AL: "Reactive mono- and di-epoxy-functionalized poly(3-hydroxybutyrate)s. Synthesis and characterization", POLYMER DEGRADATION AND STABILITY, 23 May 2012 (2012-05-23), pages 1861 - 1870, XP093061302, Retrieved from the Internet <URL:https://www.sciencedirect.com/science/article/pii/S0141391012001723> [retrieved on 20230705], DOI: 10.1016/j.polymdegradstab.2012.05.007**
- **BRANDL HELMUT ET AL: "Biodegradation of cyclic and substituted linear oligomers of poly(3-hydroxybutyrate)", CANADIAN JOURNAL OF MICROBIOLOGY, vol. 41, no. 13, 15 December 1995 (1995-12-15), CA, pages 180 - 186, XP093061344, ISSN: 0008-4166, Retrieved from the Internet <URL:http://dx.doi.org/10.1139/m95-185> [retrieved on 20230706], DOI: 10.1139/m95-185**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- GROBELNY ZBIGNIEW ET AL: "Ring-opening polymerization of [beta]-butyrolactone in the presence of alkali metal salts: investigation of initiation course and determination of polymers structure by MALDI-TOF mass spectrometry", POLYMER BULLETIN, SPRINGER, HEIDELBERG, DE, vol. 76, no. 10, 4 December 2018 (2018-12-04), pages 4951 - 4966, XP036867706, ISSN: 0170-0839, [retrieved on 20181204], DOI: 10.1007/S00289-018-2640-3

## Beschreibung

Die Erfindung betrifft Derivate von Poly-3-hydroxy-alkanoaten und Verfahren zu deren Herstellung.

Bei Poly-3-hydroxy-alkanoaten, im Folgenden auch PHA genannt, handelt es sich um lineare, aliphatische Polyester, die auf Grund ihres Eigenschaftsprofils bei hohen Molmassen (wasserunlöslich, UV-beständig, nicht-toxisch, biokompatibel, thermoplastisch und biologisch abbaubar) als nachhaltige Alternativen zu herkömmlichen Polymeren von besonderem Interesse sind (vgl. V. Sharma et al., Polymer 212 (2021) 123161). Man findet die hochmolekularen Vertreter (50 kDa - 1.000 kDa) als Speicherstoff in einer Vielzahl von Mikroorganismen, aus denen sie auch in technischem Umfang isoliert werden können. Insbesondere die am häufigsten vorkommenden Poly-3-hydroxy-butyrate, im Folgenden auch PHB genannt, können auf Grund ihrer Materialeigenschaften als biologisch abbaubarer und biokompatibler Ersatz für herkömmliche, erdölbasierte Kunststoffe wie Polypropylen insbesondere als Verpackungsmaterial für Nahrungsmittel, in der Landwirtschaft und in biomedizinischen Anwendungen eingesetzt werden.

Die Nachteile der biotechnologischen Herstellverfahren liegen jedoch in einer ungünstigen Raum-Zeit-Ausbeute, da das Bakterienwachstum natürlichen Grenzen unterliegt und somit die Ausbeuten beschränkt, sowie an teils komplexen Ausgangsverbindungen. Herstellkosten, die dem 15-fachen vergleichbarer, erdölbasierter Produkte entsprechen, erschweren darüber hinaus eine Vermarktung.

Dies betrifft nicht nur die hochmolekularen PHA-Vertreter, sondern besonders die fermentativ nicht zugänglichen, niedermolekularen Polymere bzw. Oligomere mit Molmassen im Bereich um 1.000 g/mol. Sie eignen sich auf Grund ihrer Eigenschaften (nicht-toxisch, biologisch abbaubar, biokompatibel) unter anderem zur chemischen Modifizierung von pharmazeutischen Wirkstoffen (s. G. Adamus et al., Polymers 13 (2021) 4365) oder Pestiziden zur kontrollierten verzögerten Wirkstofffreisetzung (vgl. I. Kwiecień et al., PLoS ONE, DOI:10.1371/journal.pone.0120149). Daneben wurde auch kürzlich eine intrinsische, antimikrobielle Wirksamkeit carboxyfunktioneller Oligomere festgestellt (s. L. Ma et al., Macromol. Biosci. 19 (2019) 1800432).

Ihre Herstellung kann durch Abbau von biologisch gewonnenem, hochmolekularem PHB mit Alkoholen, Diolen oder Carbonsäuren erfolgen, was jedoch aufwändig und mit hohen Kosten verbunden ist: Zuerst muss das hochmolekulare PHB biotechnologisch hergestellt, isoliert und gereinigt werden, dann erfolgt im zweiten Schritt der chemische Abbau zu kleineren Bruchstücken, der ebenfalls mit aufwändigen Aufarbeitungsschritten einhergeht (vgl. M. A. Abdelwahab et al., International Journal of Biological Macromolecules 122 (2019) 793). Zudem sind Produkte mit freien OH- oder NH-Funktionen an nur einer Endgruppe sowie A-B-A Block-Copolymere über diesen Weg nicht in einem Schritt gezielt herstellbar.

Es wurde daher intensiv versucht, diese kurzkettigen, einseitig funktionalisierten PHAs chemisch über eine Ringöffnungspolymerisation von gespannten β-Lactonen gezielt herzustellen.

Insbesondere die anionische Ringöffnungspolymerisation von β-Butyrolacton zu PHB ist in der Literatur vielfach beschrieben, da kommerziell verfügbare Nukleophile als Initiatoren eingesetzt werden können.

Es zeigte sich bei den meisten Ringöffnungspolymerisationen mit β-Butyrolacton, dass nicht nur das als Starter eingesetzte Nukleophil am Kettenanfang des entstandenen Polymers nachgewiesen werden kann, sondern bis zu 100% Crotonsäureesterreste, deren Bildung auf basisch induzierte Umlagerungen am Polymer oder am Monomer zurückgeführt werden kann (s. I. Kwiecień et al., DOI:10.1080/15685551.2013.840505.) sowie freie Crotonsäure (vgl. A. Duda, Journal of Polymer Science: Part A: Polymer Chemistry 30 (1992) 21), was die Aufreinigung des Polymers erheblich erschwert.

Die anionische Ringöffnungspolymerisation erfordert daher eine hohe Reinheit der Edukte, weshalb technische Qualitäten der Monomere aufwändig vorbehandelt werden müssen, sowie den Ausschluss von Feuchtigkeit und lange Reaktionszeiten. Die bevorzugt als Initiator eingesetzten Kaliumsalze benötigen außerdem eine Aktivierung durch hochpreisige und gesundheitsschädliche Komplexbildner wie 18-Krone-6 oder Cryptanden (s. Z. Grobelny et al., Polymer Bulletin 76 (2019) 4951), die nach der Umsetzung ebenfalls schwierig abzutrennen sind.

Die Raum-Zeit-Ausbeute wird zusätzlich durch die Notwendigkeit des Einsatzes von Lösungsmitteln wie THF oder DMSO verringert.

Alle diese Faktoren sind von Nachteil bei einer gezielten wie wirtschaftlichen Herstellung oligomerer bzw. niedermolekularer PHA-Derivate, welche an dem einen Kettenende eine Ester-Einheit als funktionelle Gruppe und an dem anderen Kettenende eine weitere funktionelle Gruppe aufweisen. Diese sind als biologisch abbaubare Additive in unterschiedlichsten Anwendungen von Interesse wie Kosmetik, Haar-, Textil-, Lederbehandlung, als Weichmacher für Polymere sowie als Basis von A-B-A-Blockcopolymeren. Sie können auch als Intermediate für vernetzbare Polymere dienen.

Die Selektivität lässt sich verbessern, wenn von vornherein ein Salz der Crotonsäure als Initiator einsetzt wird. Bereits beschrieben wurde der Einsatz von wasserfreiem Kaliumcrotonat, welches allerdings aufwändig hergestellt werden muss und zur Aktivierung den Komplexbildner 18-Krone-6 sowie THF als Lösungsmittel benötigt (s. M. Michalak et al., Polymer Degradation and Stability 97 (2012) 1861). Dabei entsteht ein PHB-Polymer, dessen eines Ende eine Crotonsäureester-Einheit bildet und das am anderen Ende eine Kaliumcarboxylatfunktion trägt. Die Kaliumcarboxylat-Endgruppe ist zwar über eine nukleophile Substitution derivatisierbar, aber man ist bei der Wahl der nukleophilen Reaktionspartner stark eingeschränkt.

Üblich wäre eine Derivatisierung des Polymers an der freien Carbonsäurefunktion beispielsweise nach Freisetzung aus dem Kaliumsalz mit einer Säure entweder über eine Veresterung mit einem Alkohol oder die Amidierung mit einem Amin. Unter den typischen Reaktionsbedingungen für diese Umsetzungen besteht allerdings die Gefahr, dass auch die Polyesterkette abgebaut wird (s. hierzu auch Vergleichsbeispiel 1).

Für eine Derivatisierung der weniger reaktiven Crotonester-Endgruppe bietet sich vorzugsweise die Epoxidierung an. Diese ist jedoch langwierig und benötigt meta-Chlorperbenzoesäure als teures Epoxidierungsreagenz im Überschuss, was eine aufwändige Reinigung nach sich zieht (vgl. M. Michalak et al., Polymer Degradation and Stability 97 (2012) 1861). Die reaktiven Epoxyfunktionen eignen sich zwar für weitere Umsetzungen, jedoch sind diese epoxidierten PHA-Derivate auf Grund des aufwändigen und damit unwirtschaftlichen Zugangs und der eingeschränkten Folgereaktionen als Intermediate zur gezielten Derivat-Herstellung für industrielle Prozesse ungeeignet.

Die selektive Herstellung von einseitig, monofunktionell substituierten PHA-Vertretern mit einer Carbonsäureestergruppe war deshalb ein lohnendes Ziel, zu dem es bislang noch keinen wirtschaftlichen Weg gab.

Es bestand deshalb die Aufgabe, ein Verfahren zur Herstellung von niedermolekularen Poly-3-hydroxyalkanoat-Derivaten zu finden, die dadurch charakterisiert sind, dass sie an dem einen Kettenende eine Ester-Einheit als funktionelle Gruppe und an dem anderen Kettenende eine weitere funktionelle Gruppe aufweisen, und welches die vorgenannten Nachteile nicht aufweist.

Diese Aufgabe wird durch die vorliegende Erfindung gelöst.

Gegenstand der Erfindung ist ein Verfahren zur Herstellung von Poly-3-hydroxy-alkanoaten der allgemeinen Formel VI, welche an dem einen Kettenende eine Ester-Einheit als funktionelle Gruppe und an dem anderen Kettenende eine weitere funktionelle Gruppe aufweisen, umfassend die folgenden Schritte:

**Schritt 1: Ringöffnungspolymerisation,** bei der mindestens ein β-Lacton der allgemeinen Formel I mit mindestens einer ungesättigten Carbonsäure der allgemeinen Formel II in Anwesenheit mindestens eines heterogenen Katalysators zu dem Poly-3-hydroxy-alkanoat der allgemeinen Formel III umgesetzt wird,

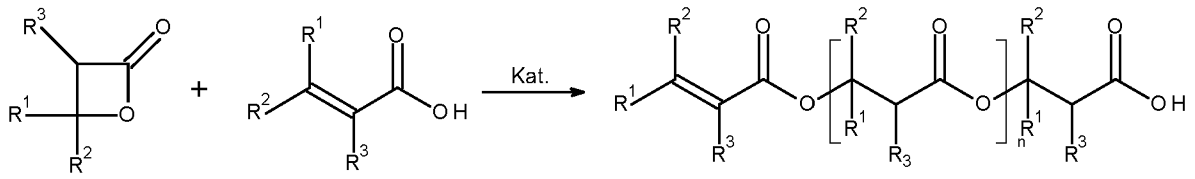


I  II  III

**Schritt 2: Chlorierung,** bei der das Poly-3-hydroxy-alkanoat der allgemeinen Formel III mit mindestens einem Chlorierungsmittel Cl umgesetzt wird, so dass das Poly-3-hydroxy-alkanoat-Carbonsäurechlorid der allgemeinen Formel IV entsteht

III 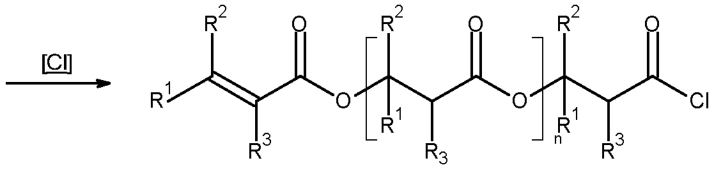


IV

und

**Schritt 3: Derivatisierung,** bei der das Poly-3-hydroxy-alkanoat-Carbonsäurechlorid der allgemeinen Formel IV mit mindestens einer mindestens eine funktionelle Gruppe X-Y aufweisenden Verbindung der allgemeinen Formel V zu dem Poly-3-hydroxy-alkanoat der allgemeinen Formel VI unter Abspaltung von Y-Cl umgesetzt wird,

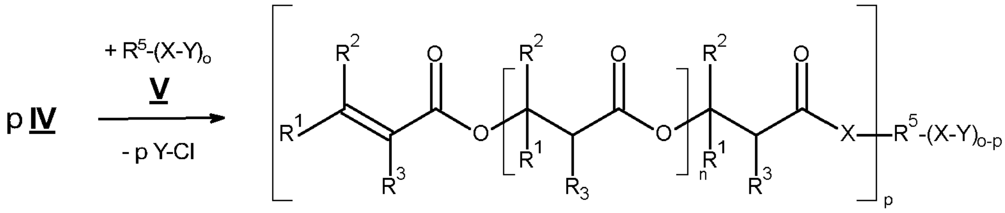


VI

wobei

**$R^1$, $R^2$, $R^3$** unabhängig voneinander ein Wasserstoffatom, ein Halogenatom, einen linearen, (bi)cyclischen oder verzweigten substituierten oder unsubstituierten $C_1$ - $C_{18}$ Kohlenwasserstoffrest, gegebenenfalls unterbrochen durch Heteroatome ausgewählt aus O, S oder N, bedeuten, wobei die Reste **$R^1$, $R^2$, $R^3$** auch paarweise verbunden als Bestandteil cyclischer Strukturen vorliegen können,
**$R^5$** einen linearen, verzweigten oder (bi)cyclischen, gesättigten oder ungesättigten, monomeren oder polymeren, substituierten oder unsubstituierten Kohlenwasserstoffrest mit 1 - 100 Kohlenstoffatomen bedeutet,
**X** -O- oder -N**$R^{11}$**- bedeutet,
wobei
**$R^{11}$** unabhängig voneinander ein Wasserstoffatom oder einen linearen, cyclischen oder verzweigten, substituierten oder unsubstituierten Alkylrest mit 1 bis 18 Kohlenstoffatomen oder einen Arylrest mit 6 bis 18 Kohlenstoffatomen oder einen Rest der allgemeinen Formel -C(=O)-**$R^{12}$** bedeutet,
wobei
**$R^{12}$** ein Wasserstoffatom oder einen linearen, cyclischen oder verzweigten, substituierten oder unsubstituierten Alkylrest mit 1 bis 18 Kohlenstoffatomen oder einen Arylrest mit 6 bis 18 Kohlenstoffatomen oder einen primären, sekundären oder tertiären Aminrest, -N**$R^8R^9$** bedeutet,
wobei
**$R^8$** und **$R^9$** unabhängig voneinander ein Wasserstoffatom oder einen Kohlenwasserstoffrest mit 1 bis 6 Kohlenstoffatomen bedeuten und die beiden Reste **$R^8$** und **$R^9$** paarweise verbunden als Bestandteil cyclischer Strukturen vorliegen können,
**Y** ein Wasserstoffatom, einen Metallrest oder einen metallhaltigen Rest bedeutet, wobei das Metall ausgewählt ist aus den Alkalimetallen, den Erdalkalimetallen, Silicium, Titan, Zink, Zinn, Eisen, Mangan oder Kupfer,
**n** 4 bis 1.400 bedeutet,
**o** 1 bis 16 bedeutet
und
**p** $\leq$ **o** bedeutet.

Um die Seitenzahl der Beschreibung der vorliegenden Erfindung nicht zu umfangreich zu gestalten, werden im Folgenden nur die bevorzugten Ausführungsformen der einzelnen Merkmale aufgeführt.

Der fachkundige Leser soll diese Art der Offenbarung aber explizit so verstehen, dass damit auch jede Kombination aus unterschiedlichen Bevorzugungsstufen explizit offenbart und explizit gewünscht ist.

In einer bevorzugten Ausführungsform können $R^1$ und $R^2$ an den Doppelbindungen vertauscht als E- oder Z-Isomere oder Gemische daraus vorliegen.

Vorzugsweise bedeutet **$R^5$** einen linearen, verzweigten oder (bi)cyclischen, gesättigten oder ungesättigten, monomeren oder polymeren, substituierten oder unsubstituierten Kohlenwasserstoffrest mit 3 - 100 Kohlenstoffatomen.

Bevorzugt bedeutet die funktionelle Gruppe X-Y -OH, -NH$R^{11}$, ein Li-, Na-, K-, Mg- oder Ca-Alkoholat oder ein Sauerstoff-gebundenes Silyl, besonders bevorzugt -OH, ein Na- oder K-Alkoholat oder ein Sauerstoff-gebundenes Trialkylsilyl, wobei der Alkylrest 1 bis 5 Kohlenstoffatome umfasst, und ganz besonders bevorzugt -OH oder ein Sauerstoff-gebundenes Trimethylsilyl.

Vorzugsweise bedeutet **n** 4 bis 100 und ganz besonders bevorzugt 4 bis 20.

Überraschend wurde gefunden, dass β-Lactone technischer Qualität an heterogen basischen Katalysatoren in Poly-3-hydroxy-alkanoate der allgemeinen Formel III umgesetzt werden können. Ein β-Lacton technischer Qualität bedeutet, dass als Hauptverunreinigung die zu dem β-Lacton isomere Carbonsäure vorliegt. Im Allgemeinen beträgt die Reinheit des β-Lactons $\geq$ 98 Gew.-% und bevorzugt $\geq$ 95 Gew.-%. Als β-Lactone der allgemeinen Formel I wird ganz besonders bevorzugt ein β-Lacton technischer Qualität mit einer Reinheit von mindestens $\geq$ 90 Gew.-% eingesetzt.

Aus der Struktur des resultierenden Polymers kann geschlossen werden, dass vorzugsweise als Initiator für die Ringöffnungspolymerisation in Schritt 1 mindestens eine ungesättigte Carbonsäure der allgemeinen Formel II fungiert, die einem Isomer des β-Lactons der allgemeinen Formel I entspricht. Diese wird während der Polymerisation durch

Umlagerung aus dem β-Lacton der allgemeinen Formel I gebildet oder liegt bereits zu Beginn als typischer Nebenbestandteil in technischen Qualitäten der β-Lactone der allgemeinen Formel I vor. Sie wird allein durch den heterogenen Katalysator aktiviert. Dass dafür weder ein Lösungsmittel noch ein aktivierender Komplexbildner notwendig sind, war nicht vorhersehbar.

Das erfindungsgemäße Verfahren beinhaltet darüber hinaus in Schritt 2 die nachfolgende Umwandlung der Carboxy-Endgruppe des Poly-3-hydroxy-alkanoates der allgemeinen Formel III in eine reaktive Carbonsäurechlorid-Funktion. Auch hierbei war überraschend, dass diese Reaktion ohne nennenswerte Nebenreaktionen oder nennenswerten Polymerabbau erfolgt.

Ein weiterer Schritt des erfindungsgemäßen Verfahrens ist Schritt 3, die Umwandlung der reaktiven Carbonsäurechlorid-Endgruppe in dem Poly-3-hydroxy-alkanoat-Carbonsäurechlorid der allgemeinen Formel IV, vorzugsweise mit einer OH-Gruppe oder einer NH-Gruppe in ein Folgeprodukt, vorzugsweise einen Ester oder ein Amid. Vorzugsweise wird insbesondere bei der Umsetzung mit OH-Verbindungen kein Lösungsmittel eingesetzt und der gebildete Chlorwasserstoff direkt aus dem Gleichgewicht durch Anlegen von Vakuum entfernt. Er kann damit einem Recycling zugeführt werden, was den Prozess ressourcenschonend gestaltet.

Das erfindungsgemäße Verfahren umfasst vorzugsweise demnach drei Schritte, bei dem im Batchbetrieb mindestens zwei aufeinanderfolgende Schritte ohne Zwischenaufreinigung im selben Reaktionsgefäß oder alle Schritte in einem semi- oder vollkontinuierlichen Verfahren in nacheinander geschalteten Reaktoren durchgeführt werden, was einen weiteren, insbesondere wirtschaftlichen Vorteil darstellt.

Das Verfahren ist des Weiteren dadurch gekennzeichnet, dass die Schritte 1 bis 3 bevorzugt lösungsmittelfrei durchgeführt werden. Etwaige Verunreinigungen der Edukte oder Zwischenprodukte durch Lösungsmittelreste können dabei enthalten sein.

Ein weiterer Gegenstand der Erfindung sind Poly-3-hydroxy-alkanoat-Carbonsäurechloride der allgemeinen Formel,

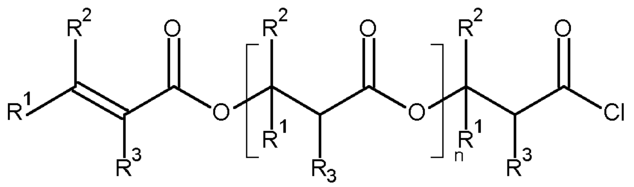

**IV**

wobei

> **$R^1$, $R^2$, $R^3$** unabhängig voneinander ein Wasserstoffatom, ein Halogenatom, einen linearen, (bi)cyclischen oder verzweigten, substituierten oder unsubstituierten $C_1$ - $C_{18}$ Kohlenwasserstoffrest, gegebenenfalls unterbrochen durch Heteroatome ausgewählt aus O, S oder N, bedeuten, wobei die Reste **$R^1$, $R^2$, $R^3$** auch paarweise verbunden als Bestandteil cyclischer Strukturen vorliegen können
> und
> **n** 4 bis 1.400 bedeutet.

Ein weiterer Gegenstand der Erfindung sind Poly-3-hydroxy-alkanoate der allgemeinen Formel,

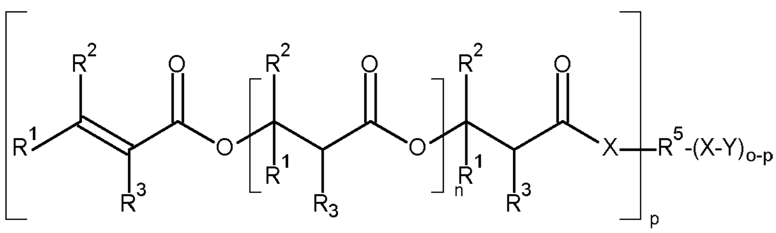

**VI**

wobei

> **$R^1$, $R^2$, $R^3$** unabhängig voneinander ein Wasserstoffatom, ein Halogenatom, einen linearen, (bi)cyclischen oder verzweigten, substituierten oder unsubstituierten $C_1$ - $C_{18}$ Kohlenwasserstoffrest, gegebenenfalls unterbrochen

durch Heteroatome ausgewählt aus O, S oder N, bedeuten, wobei die Reste **$R^1$, $R^2$, $R^3$** auch paarweise verbunden als Bestandteil cyclischer Strukturen vorliegen können,

**$R^5$** einen linearen, verzweigten oder (bi)cyclischen, gesättigten oder ungesättigten, monomeren oder polymeren, substituierten oder unsubstituierten Kohlenwasserstoffrest mit 1 - 100 Kohlenstoffatomen bedeutet,

**X** -O- oder -N**$R^{11}$**- bedeutet,

wobei

**$R^{11}$** unabhängig voneinander ein Wasserstoffatom oder einen linearen, cyclischen oder verzweigten, substituierten oder unsubstituierten Alkylrest mit 1 bis 18 Kohlenstoffatomen oder einen Arylrest mit 6 bis 18 Kohlenstoffatomen oder einen Rest der allgemeinen Formel -C(=O)-**$R^{12}$** bedeutet,

wobei

**$R^{12}$** ein Wasserstoffatom oder einen linearen, cyclischen oder verzweigten, substituierten oder unsubstituierten Alkylrest mit 1 bis 18 Kohlenstoffatomen oder einen Arylrest mit 6 bis 18 Kohlenstoffatomen oder einen primären, sekundären oder tertiären Aminrest, -N**$R^8R^9$** bedeutet,

wobei

**$R^8$** und **$R^9$** unabhängig voneinander einen Wasserstoffrest oder einen Kohlenwasserstoffrest mit 1 bis 6 Kohlenstoffatomen bedeuten und die beiden Reste **$R^8$** und **$R^9$** paarweise verbunden als Bestandteil cyclischer Strukturen vorliegen können,

**Y** ein Wasserstoffatom, einen Metallrest oder einen metallhaltigen Rest bedeutet, wobei das Metall ausgewählt ist aus den Alkalimetallen, den Erdalkalimetallen, Silicium, Titan, Zink, Zinn, Eisen, Mangan oder Kupfer,

**n** 4 bis 1.400 bedeutet,

**o** 1 bis 16 bedeutet

und

$p < o$ bedeutet.

In den erfindungsgemäßen Poly-3-hydroxy-alkanoaten der allgemeinen Formel VI bedeutet

**$R^5$** bevorzugt einen linearen, verzweigten oder (bi)cyclischen, gesättigten oder ungesättigten, monomeren oder polymeren, substituierten oder unsubstituierten Kohlenwasserstoffrest mit 3 - 100 Kohlenstoffatomen

und

die funktionellen Gruppe X-Y vorzugsweise -OH, -NH$R^{11}$, ein Li-, Na-, K-, Mg- oder Ca-Alkoholat oder ein Sauerstoff-gebundenes Silyl, besonders bevorzugt -OH, ein Na- oder K-Alkoholat oder ein Sauerstoff-gebundenes Trialkylsilyl, wobei der Alkylrest 1 bis 5 Kohlenstoffatome umfasst, und ganz besonders bevorzugt - OH oder ein Sauerstoff-gebundenes Trimethylsilyl.

In den Poly-3-hydroxy-alkanoat-Carbonsäurechloriden der allgemeinen Formel IV oder den Poly-3-hydroxy-alkanoaten der allgemeinen Formel VI bedeutet **n** bevorzugt 4 bis 100 und besonders bevorzugt 4 bis 20.

Die Poly-3-hydroxy-alkanoat-Carbonsäurechloride der allgemeinen Formel IV und die Poly-3-hydroxy-alkanoate der allgemeinen Formel VI sowie das erfindungsgemäße Verfahren zu deren Herstellung werden im Folgenden detaillierter beschrieben:

**Schritt 1: Ringöffnungspolymerisation**

Ein β-Lacton der allgemeinen Formel

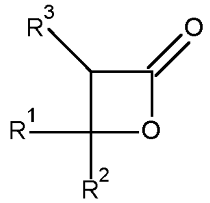

**I**

oder ein Gemisch verschiedener β-Lactone, welche kommerziell verfügbar oder nach bekannten Methoden zugänglich sind (s. z.B. WO2010118128 oder WO2022143914),

wobei

**$R^1$, $R^2$, $R^3$** unabhängig voneinander ein Wasserstoffatom, ein Halogenatom, vorzugsweise F, Cl und Br, besonders

bevorzugt F und Cl, einen linearen, (bi)cyclischen oder verzweigten, substitutierten oder unsubstituierten $C_1$ - $C_{18}$ Kohlenwasserstoffrest, bevorzugt ausgewählt aus Alkyl-, Alkenyl-, Alkinyl-, Alkylaryl-, Arylalkyl- oder Arylresten, gegebenenfalls unterbrochen durch Heteroatome ausgewählt aus O, S, oder N, bedeuten
und wobei
**$R^1$, $R^2$, $R^3$** auch paarweise verbunden als Bestandteil cyclischer Strukturen vorliegen können.

In einer bevorzugten Ausführungsform können **$R^1$, $R^2$, $R^3$** unabhängig voneinander ein Wasserstoffatom, ein Halogenatom, vorzugsweise F, Cl und Br, besonders bevorzugt F und Cl, einen linearen, (bi)cyclischen oder verzweigten, substitutierten oder unsubstituierten $C_1$ - $C_{18}$ Kohlenwasserstoffrest, ausgewählt aus Alkyl-, Alkenyl-, Alkinyl-, Alkylaryl-, Arylalkyl- oder Arylresten, gegebenenfalls unterbrochen durch -N**$R^4$**- bedeuten, wobei niemals 2 Heteroatome direkt benachbart sind, gegebenenfalls substituiert durch Halogenatome, Carboxyalkyl, Alkoxy- oder Aminoreste,
wobei **$R^4$** einen linearen, cyclischen oder verzweigten, substituierten oder unsubstituierten Alkylrest mit 1 bis 18 Kohlenstoffatomen oder einen Arylrest mit 6 bis 18 Kohlenstoffatomen bedeutet.

Nicht abschließende Beispiele für $C_1$-$C_{18}$-Kohlenwasserstoffreste **$R^1$, $R^2$, $R^3$** und **$R^4$** sind Alkylreste wie der Methyl-, Ethyl-, n-Propyl-, iso-Propyl-, n-Butyl-, 2-Butyl-, iso-Butyl-, tert.-Butyl-, n-Pentyl-, iso-Pentyl-, neo-Pentyl-, tert.-Pentylrest, Hexylreste wie der n-Hexylrest, Heptylreste wie der n-Heptylrest, Octylreste wie der n-Octylrest und iso-Octylreste wie der 2,2,4-Trimethylpentylrest, Nonylreste wie der n-Nonylrest, Decylreste wie der n-Decylrest; Cycloalkylreste wie Cyclopentyl-, Cyclohexyl-, 4-Ethylcyclohexyl-, Cycloheptylreste, Norbornylreste und Methylcyclohexylreste oder auch Alkenylreste wie der Vinyl-, 2-Propen-2-yl-, Allyl-, 3-Buten-1-yl-, 5-Hexen-1-yl-, 10-Undecen-1-yl, Cycloalkenylreste wie der 2-Cyclohexenyl-, 3-Cyclohexenyl-, Cyclopentadienylrest, 2-(Cyclohex-3-en-1-yl)ethyl, Arylreste wie der Phenyl-, Biphenylyl-, Naphthylrest, Alkarylreste wie o-, m-, p-Tolylreste und Phenethylreste wie 2-Phenylethyl-, 1-Phenylethylrest und Aralkylreste wie der Benzylrest, Alkylenylreste wie der 1,2-Ethylen-, der 1,3-Propylen-, der 1,4-Butylenrest, Arylenreste wie der 1,2-Phenylenrest.

Vorzugsweise stehen die Reste **$R^1$, $R^2$, $R^3$** und **$R^4$** für einen linearen oder verzweigten, substituierten oder unsubstituierten Alkylrest mit 1 bis 8 Kohlenstoffatomen, besonders bevorzugt sind der Methyl-, Ethyl-, 1-Propyl-, 2-Propyl-, Phenyl-, Vinyl-, n-Hexyl-, 1-Phenylethyl-, 2-Phenylethenylrest, ganz besonders der Propylrest und der Methylrest, insbesondere der Methylrest, ganz besonders bevorzugt steht **$R^1$** für den Methyl-, den -1-Propyl- oder den 2-Propylrest und die Reste **$R^2$, $R^3$** für ein Wasserstoffatom.

Insbesondere handelt es sich bei dem β-Lacton der allgemeinen Formel I um β-Butyrolacton.

Das β-Lacton der allgemeinen Formel I wird vorzugsweise in Gegenwart von 0,1 bis 20 Gewichtsprozent, besonders bevorzugt 0,1 bis 10 Gewichtsprozent, ganz besonders bevorzugt 0,5 bis 5 Gewichtsprozent, insbesondere 1 bis 3 Gewichtsprozent bezogen auf das Gewicht der Edukte eines festen, basischen Katalysators umgesetzt. Der Katalysator wird vorzugsweise ausgewählt aus der Gruppe der Alkalimetalloxide, -hydroxide, -carbonate und - hydrogencarbonate oder Alkalimetallfluoride, gegebenenfalls auf Trägermaterialien wie basischem, saurem oder neutralem Aluminiumoxid, Titandioxid, Zirkoniumoxid, gefällter oder pyrogener Kieselsäure wie beispielsweise beschrieben in US5223595 A (= korrespondierende DE 41 16014 A1), oder Erdalkalimetalloxide oder -hydroxide. Auf Grund ihrer kommerziellen Verfügbarkeit und Reaktivität werden als Katalysator bevorzugt Kaliumsalze, besonders bevorzugt wasserfreies Kaliumcarbonat und Kaliumfluorid*Aluminiumoxid im Molverhältnis 1:1 und ganz besonders bevorzugt wasserfreies Kaliumcarbonat eingesetzt.

Das β-Lacton der allgemeinen Formel I wird vorzugsweise unter Zusatz von 0 - 10 Molprozent, besonders bevorzugt 0 - 5 Molprozent, insbesondere 0 - 1 Molprozent bezogen auf die eingesetzte Menge an β-Lacton der allgemeinen Formel I, eines Initiators für die Ringöffnungspolymerisation, ausgewählt aus einer Carbonsäure mit 1 bis 22 Kohlenstoffatomen, vorzugsweise der dem eingesetzten β-Lacton der allgemeinen Formel I entsprechenden isomeren ungesättigten Carbonsäure der allgemeinen Formel II, beispielsweise E-Crotonsäure oder E-2-Hexensäure, Ameisensäure, Essigsäure, Benzoesäure, Zimtsäure, Acrylsäure, Methacrylsäure, Sorbinsäure, Octansäure, Nonansäure, Undecensäure, Laurinsäure, Ölsäure, Linolsäure, Linolensäure, Palmitinsäure, Stearinsäure, Erucasäure, wobei das Molverhältnis aus β-Lacton der allgemeinen Formel I und gegebenenfalls zugesetztem Initiator vorzugsweise so gewählt wird, dass der gewünschte Polymerisationsgrad bzw. das gewünschte Molgewicht an Poly-3-hydroxy-alkanoat der allgemeinen Formel III bei der Ringöffnungspolymerisation erreicht und so lange umgesetzt wird, bis der gewünschte Umsatz erreicht ist. Das Umsatzziel wird vorzugsweise über die Reaktionsbedingungen wie Reaktionsdauer, -temperatur, Art des Katalysators, Katalysatorkonzentration oder Initiatorkonzentration dahingehend optimiert, dass bei maximaler Ausbeute an Poly-3-hydroxy-alkanoat der allgemeinen Formel III ein Minimum unerwünschter Nebenprodukte, insbesondere die aus dem β-Lacton der allgemeinen Formel I gebildeten isomeren ungesättigten Carbonsäuren der allgemeinen Formel II wie beispielsweise Crotonsäure aus β-Butyrolacton, auftritt. Dies lässt sich in Vorversuchen durch Einsatz analytischer Methoden, vorzugsweise IR-, Raman-, NMR-, MALDI-TOF-, oder ESI-MS-Spektroskopie oder LC- bzw. SEC-Chromatographie einfach ermitteln.

Die Umsetzung wird vorzugsweise im Temperaturbereich zwischen 0 und 200°C, besonders bevorzugt zwischen 20 und 150°C, insbesondere im Bereich des Siedepunkts des Reaktionsgemischs und beim Druck der umgebenden Atmosphäre, gegebenenfalls auch unter einem höheren oder niedrigeren Druck, durchgeführt. Aus Sicherheitsgründen und zum Ausschluss von Feuchtigkeit wird vorzugsweise unter trockenem Inertgas wie Stickstoff oder Argon umgesetzt.

Der Abbruch der Polymerisation kann durch Entfernen des nicht umgesetzten β-Lactons der allgemeinen Formel I durch Destillation oder Abkühlen der Reaktionsmischung oder Abtrennung des festen Katalysators durch Filtration, Zentrifugieren oder Dekantieren beispielsweise in einem Bypass des Reaktionsgefäßes oder durch dessen chemische Deaktivierung wie beispielsweise Neutralisation erfolgen.

Eine kontinuierliche Reaktionsführung bietet sich an, da die Reaktionsmischung beispielsweise in einem Schleifenreaktor über ein Katalysator-Festbett so lange umgepumpt werden kann, bis der gewünschte Umsatz erreicht ist.

Die mittleren Molmassen $\mathbf{M_w}$ der Poly-3-hydroxy-alkanoate der allgemeinen Formel III aus Schritt 1 des erfindungsgemäßen Verfahrens liegen vorzugsweise im Bereich von 300 g/mol bis 100.000 g/mol, besonders bevorzugt zwischen 300 g/mol und 30.000 g/mol, insbesondere zwischen 300 g/mol und 5.000 g/mol. Ganz besonders bevorzugt werden Molmassen, bei denen die Polymere bei Raumtemperatur flüssig sind. Je nach Molmasse der eingesetzten Monomere ergibt sich ein Wert für den Index **n** in dem Poly-3-hydroxy-alkanoat der allgemeinen Formel III von 4 bis 1.400. Für Poly-3-hydroxy-alkanoate der allgemeinen Formel III bedeutet der Index **n** bevorzugt 4 bis 100 und liegt besonders bevorzugt im Bereich von 4 bis 20. Die Indizes **n** der Poly-3-hydroxy-alkanoat-Carbonsäurechloride der allgemeinen Formel IV und Poly-3-hydroxy-alkanoate der allgemeinen Formel VI liegen vorzugsweise im selben Bereich oder geringfügig niedriger, da es bei den Folgeschritten zu einem geringen Abbau der Polymerketten kommen kann.

Die mittleren Molmassen $\mathbf{M_w}$ können beispielweise über NMR-Spektroskopie, SEC, GPC, LC, MALDI-TOF oder ESI-MS bestimmt werden.

**Schritt 2: Chlorierung**

Die in Schritt 1 erhaltenen Poly-3-hydroxy-alkanoate der allgemeinen Formel III werden mit einem typischen Chlorierungsmittel Cl für Carbonsäuren versetzt und bis zur vollständigen Umwandlung der Carbonsäuregruppe an einem Ende des Poly-3-hydroxy-alkanoats der allgemeinen Formel III in die entsprechende Carbonsäurechloridgruppe umgesetzt.

Als Chlorierungsmittel Cl werden vorzugsweise Thionylchlorid, Phosgen oder Phosphor(V)chlorid und besonders bevorzugt Thionylchlorid eingesetzt. Vorteilhaft ist dessen relativ geringe Toxizität und die schonende Chlorierung unter Bildung ausschließlich leicht flüchtiger Spaltprodukte $SO_2$ und HCl, die beide einem Recycling zugeführt werden können.

Zur Aufarbeitung des Poly-3-hydroxy-alkanoat-Carbonsäurechlorids der allgemeinen Formel IV genügt ein Entflüchtigen oder Abdestillieren flüchtiger Bestandteile. Außerdem muss der heterogene Katalysator vorher nicht abgetrennt werden. Im Fall eines Alkalimetalloxids, -carbonats oder -hydroxids wird der Katalysator durch das Chlorierungsmittel neutralisiert und damit deaktiviert. Dies muss gegebenenfalls bei der Stöchiometrie des Chlorierungsmittels Cl zu Poly-3-hydroxy-alkanoat der allgemeinen Formel III berücksichtigt werden. Eventuell gebildete, in der Regel feste Neutralisationsprodukte des Katalysators müssen vor dem nächsten Schritt der Derivatisierung nicht abgetrennt werden, da sie sich inert verhalten. Ihre Abtrennung erfolgt vorzugsweise durch Filtration, Sedimentation oder Zentrifugieren erst nach Schritt 3 der Derivatisierung. Vorteilhaft ist dabei, dass die Katalysatoren beziehungsweise deren deaktivierte Folgeprodukte salzartig sind und sich damit als Feststoffe leicht abtrennen lassen, was eine hohe Reinheit der erfindungsgemäßen Poly-3-hydroxy-alkanoate der allgemeinen Formel VI ermöglicht. Wasserlösliche Nebenprodukte können auch durch einen Waschvorgang mit Wasser vom Polymer abgetrennt werden.

Typische Nebenbestandteile wie die zum eingesetzten β-Lacton der allgemeinen Formel I isomere ungesättigte Carbonsäure der allgemeinen Formel II oder andere nicht vollständig umgesetzte, als Initiatoren eingesetzte Carbonsäuren werden bei der Chlorierung ebenfalls in die Carbonsäurechloride überführt. Diese lassen sich auf Grund der niedrigeren Siede- und Schmelzpunkte destillativ wesentlich schonender abtrennen als die jeweiligen freien Carbonsäuren.

Vorzugsweise wird das Chlorierungsmittel Cl äquimolar bis zum 5-fachen molaren Überschuss zu dem eingesetzten Poly-3-hydroxy-alkanoat der allgemeinen Formel III aus Schritt 1 des erfindungsgemäßen Verfahrens eingesetzt. Besonders bevorzugt wird ein 1,1 bis 3-facher molarer Überschuss verwendet, insbesondere ein 1,1 bis 2-facher molarer Überschuss bezogen auf den Carbonsäureanteil in der Reaktionsmischung, vorzugsweise bestehend aus Poly-3-hydroxy-alkanoat der allgemeinen Formel III und gegebenenfalls nicht umgesetzter oder aus dem β-Lacton der allgemeinen Formel II gebildeter Carbonsäure.

Das Chlorierungsmittel Cl kann vorzugsweise von Anfang an komplett zugegeben oder bis zur vollständigen Umsetzung permanent zudosiert werden. Feste oder gasförmige Chlorierungsmittel können dafür gelöst in einem Lösungsmittel eingesetzt werden. Thionylchlorid wird vorzugsweise von Beginn an komplett zugesetzt.

Die Chlorierungsmittel, vorzugsweise Thionylchlorid, können in technischer Qualität eingesetzt werden. Technische Qualität umfasst Reinheiten von im Allgemeinen ≥ 98 Gew.-%, bevorzugt ≥ 95 Gew.-% und ganz besonders bevorzugt ≥ 90 Gew.-%. Nicht umgesetzte Anteile an Chlorierungsmittel Cl können einfach abdestilliert und wiederverwendet werden. Insofern kann ein Überschuss Thionylchlorid auch gleichzeitig zur Herabsetzung der Viskosität der Reaktionsmischung in Schritt 2 eingesetzt werden.

Die Reaktionstemperaturen liegen vorzugsweise in dem für diese Reaktionen typischen Bereich zwischen -10°C und 100°C, besonders bevorzugt zwischen 20°C und 70°C insbesondere zwischen 20°C und 50°C. Es können auch Temperaturrampen gefahren werden, indem vorzugsweise während der Umsetzung die Temperatur stufenweise oder kontinuierlich erhöht wird, um die Reaktion bei möglichst geringem Anteil Nebenreaktionen gegen Ende zu beschleunigen. Vorzugsweise erfolgt die Umsetzung unter dem Atmosphärendruck der Umgebung oder unter vermindertem Druck, um die flüchtigen Nebenprodukte rasch aus der Reaktionsmischung zu entfernen und Nebenreaktionen zu minimieren oder zu vermeiden. Für den Fall, dass **Y** ein Wasserstoffatom bedeutet, ist insbesondere die HCl-Addition an die Doppelbindung der ungesättigten Esterfunktion eine typische Nebenreaktion, die zu einer 3-Chlorcarbonsäureesterendgruppe führt. Falls diese Reaktion gewünscht ist, kann die ungesättigte Esterendgruppe durch entsprechende Verlängerung der Reaktionszeit ohne Entfernung des Chlorwasserstoffs aus der Reaktionsmischung oder zusätzliches Einleiten von Chlorwasserstoffgas vollständig in die 3-Chlorcarbonsäureesterendgruppe umgewandelt werden.

Besonders bevorzugt erfolgt jedoch die Umsetzung unter vermindertem Druck, vorzugsweise bei 10 - 900 hPa, insbesondere 50 - 200 hPa. Ein Durchleiten von Inertgas wie Stickstoff, Kohlendioxid oder Argon kann ebenfalls unterstützend eingesetzt werden gegebenenfalls in Kombination mit reduziertem Druck.

Das Verfahren ist dadurch gekennzeichnet, dass Schritt 2 bevorzugt lösungsmittelfrei durchgeführt wird. Etwaige Verunreinigungen der Edukte oder Zwischenprodukte durch Lösungsmittelreste können enthalten sein.

Ein Lösungsmittel kann zum Beispiel zur Verbesserung der Durchmischung zugegeben werden. Bevorzugte Lösungsmittel besitzen gute Lösungseigenschaften, verhalten sich gegenüber den Bestandteilen der Reaktionsmischung inert, lassen sich nachträglich leicht abtrennen, beispielsweise für ein Recycling, sind kommerziell verfügbar und gesundheitlich sowie sicherheitstechnisch möglichst unbedenklich.

Nicht abschließende Beispiele dafür sind Anisol, Xylol, Alkane wie n-Heptan, n-Octan, n-Decan, Isoalkane wie Isopar® E von Exxon, Ether, wie Di-n-butylether, 2-Methyl-tetrahydrofuran, Methyl-t-butylether oder Gemische davon. Besonders bevorzugt ist Anisol.

Andere mögliche Lösungsmittel sind Toluol, Tetrahydrofuran, Chlorbenzol, N,N-Dimethylformamid, Dimethylsulfoxid, Dichlormethan oder Trichlormethan.

Das Ende der Reaktion kann entweder durch Probenahme aus der Reaktionsmischung analytisch über spektroskopische Methoden bestimmt werden, beispielsweise über $^{1}$H- oder $^{13}$C-NMR-Spektroskopie oder durch Verfolgung der Gasentwicklung, beziehungsweise der quantitativen Bestimmung der freigesetzten Spaltprodukte.

Die mittleren Molmassen $\mathbf{M_w}$ der Poly-3-hydroxy-alkanoat-Carbonsäurechloride der allgemeinen Formel IV aus Schritt 2 des erfindungsgemäßen Verfahrens liegen vorzugsweise im Bereich der mittleren Molmassen der Poly-3-hydroxy-alkanoate der allgemeinen Formel III.

Die mittleren Molmassen $\mathbf{M_w}$ können beispielweise über NMR-Spektroskopie, SEC, GPC, LC, MALDI-TOF oder ESI-MS bestimmt werden.

### Schritt 3: Derivatisierung

Die in Schritt 2 des erfindungsgemäßen Verfahrens erhaltenen Poly-3-hydroxy-alkanoat-Carbonsäurechloride der allgemeinen Formel IV werden vorzugsweise direkt im Anschluss mit mindestens einer mindestens eine funktionelle Gruppe X-Y aufweisenden Verbindung der allgemeinen Formel V

$$R^5\text{-}(X\text{-}Y)_o \qquad \mathbf{V}$$

unter Abspaltung von Y-Cl umgesetzt, wobei Cl Chlor bedeutet.

$\mathbf{R^5}$ bedeutet einen linearen, verzweigten oder (bi)cyclischen, gesättigten oder ungesättigten, monomeren oder polymeren, substituierten oder unsubstituierten Kohlenwasserstoffrest mit 1 - 100 Kohlenstoffatomen, bevorzugt einen linearen, verzweigten oder (bi)cyclischen, gesättigten oder ungesättigten, monomeren oder polymeren, substituierten oder unsubstituierten Kohlenwasserstoffrest mit 3 - 100 Kohlenstoffatomen.

**X** bedeutet dabei **-O-** oder **-NR$^{11}$-,**
wobei

> $\mathbf{R^{11}}$ unabhängig voneinander ein Wasserstoffatom oder einen linearen, cyclischen oder verzweigten, substituierten oder unsubstituierten Alkylrest mit 1 bis 18 Kohlenstoffatomen oder einen Arylrest mit 6 bis 18 Kohlenstoffatomen oder einen Rest der allgemeinen Formel -C(=O)-$\mathbf{R^{12}}$ bedeutet,
> wobei

$R^{12}$ ein Wasserstoffatom oder einen linearen, cyclischen oder verzweigten, substituierten oder unsubstituierten Alkylrest mit 1 bis 18 Kohlenstoffatomen oder einen Arylrest mit 6 bis 18 Kohlenstoffatomen oder einen primären, sekundären oder tertiären Aminrest -N$R^8R^9$ bedeutet,
wobei
$R^8$ und $R^9$ unabhängig voneinander ein Wasserstoffatom oder einen Kohlenwasserstoffrest mit 1 bis 6 Kohlenstoffatomen bedeuten und die beiden Reste $R^8$ und $R^9$ paarweise verbunden als Bestandteil cyclischer Strukturen (Alkylen- oder Arylen-Reste) vorliegen können.

**Y** bedeutet dabei ein Wasserstoffatom, einen Metallrest oder einen metallhaltigen Rest, wobei das Metall ausgewählt ist aus den Alkalimetallen, vorzugsweise Lithium, Natrium, Kalium oder Cäsium, besonders bevorzugt Natrium und Kalium, den Erdalkalimetallen, vorzugsweise Magnesium oder Calcium, Silicium, Titan, Zink, Zinn, Eisen, Mangan oder Kupfer.

Bei den Metallresten ist ein Metallion vorwiegend ionisch an einen Organylrest angebunden oder koordiniert wie beispielsweise in Metallalkoholaten.

Bei den metallhaltigen Resten ist ein organischer Rest oder eine organische Verbindung direkt an ein Metallatom gebunden. Diese Verbindungen werden als Metallorganyle bezeichnet. Es zählen auch Derivate solcher Elemente zu den metallorganischen Verbindungen, die zwar im elementaren Zustand kein Metall bilden, aber eine niedrige Elektronegativität aufweisen wie zum Beispiel Silicium. Metallorganyle enthalten also mindestens ein Kohlenstoffatom und mindestens ein Metall- bzw. elektropositives Elementatom die aneinandergebunden sind. Diese Bindung ist dabei eine mehr oder weniger polare kovalente Bindung. Der Organylrest kann dabei entweder über eine Einfach-, Doppel- oder sogar Dreifachbindung an das Element gebunden sein, oder gleich mehrfach mit dem Elementatom verknüpft sein.

Bevorzugt bedeutet die funktionelle Gruppe X-Y -OH, -NH$R^{11}$, ein Li-, Na-, K-, Mg- oder Ca-Alkoholat oder ein Sauerstoff-gebundenes Silyl, besonders bevorzugt -OH, ein Na- oder K-Alkoholat oder ein Sauerstoff-gebundenes Trialkylsilyl, wobei der Alkylrest 1 bis 5 Kohlenstoffatome umfasst, und ganz besonders bevorzugt -OH oder ein Sauerstoff-gebundenes Trimethylsilyl.

Vorzugsweise können auch mehrere verschiedene Einheiten X-Y in der Verbindung der allgemeinen Formel V vorliegen oder Mischungen aus mindestens zwei Verbindungen der allgemeinen Formel V eingesetzt werden.

**o** bedeutet 1 bis 16, vorzugsweise 1 bis 8, ganz besonders bevorzugt 1 bis 3, insbesondere 1 oder 2.

Für eine vollständige Reaktion aller in der Verbindung der allgemeinen Formel V vorliegenden Gruppen X-Y werden vorzugsweise **o** Mol Poly-3-hydroxy-alkanoat-Carbonsäurechlorid der allgemeinen Formel IV pro Mol der Verbindung der allgemeinen Formel V eingesetzt, was **p = o** entspricht.

Sollen im Poly-3-hydroxy-alkanoat der allgemeinen Formel VI noch freie X-Y-Gruppen vorliegen, wird vorzugsweise die Molmenge **p** des Poly-3-hydroxy-alkanoat-Carbonsäurechlorids der allgemeinen Formel IV entsprechend kleiner gewählt. Vorzugsweise ist somit **p < o.** Steht in diesem Fall **Y** nicht für ein Wasserstoffatom und sollen im Poly-3-hydroxy-alkanoat der allgemeinen Formel VI freie X-H-Gruppen vorliegen, so können diese aus den freien X-Y-Gruppen mit einer Säure, vorzugsweise ausgewählt aus anorganischen Säuren wie Chlorwasserstoff, Schwefelsäure, Salpetersäure, ortho-Phosphorsäure, metaPhosphorsäure oder deren sauren Salze oder Ester oder organischen Säuren wie Ameisensäure, Essigsäure, Benzolsulfonsäure oder Methansulfonsäure freigesetzt werden.

$R^5$ in der allgemeinen Formel V kann vorzugsweise funktionelle Gruppen enthalten, ausgewählt aus tertiären Aminfunktionen oder deren Ammoniumsalzen, N-haltigen Heterocyclen, O-haltigen Heterocyclen, S-haltigen Heterocyclen, O,N-haltigen Heterocyclen, quaternären Ammoniumgruppen, Nitrofunktionen, Nitrilfunktionen, Ketonfunktionen, monomeren, oligomeren oder polymeren Ethereinheiten, monomeren, oligomeren oder polymeren Carbonsäureestereinheiten, Phosphon- oder Phosphorsäureestereinheiten, Reste mit Kohlenstoff-KohlenstoffDoppelbindungen oder Kohlenstoff-Kohlenstoff-Dreifachbindungen, besonders bevorzugt sind tertiäre Aminfunktionen sowie oligomere und polymere Polyethereinheiten, insbesondere tertiäre Aminreste sowie Polyethylenglykolreste.

$R^5$ bedeutet in der Verbindung der allgemeinen Formel V einen linearen, verzweigten oder (bi)cyclischen, gesättigten oder ungesättigten, monomeren oder polymeren, substituierten oder unsubstituierten Kohlenwasserstoffrest mit 1 - 100 Kohlenstoffatomen, bevorzugt mit 3 - 100 Kohlenstoffatomen.

In einer bevorzugten Ausführungsform wird $R^5$ ausgewählt aus Alkyl-, Alkenyl-, Alkinyl-, Alkylaryl-, Arylalkyl- oder Arylresten, gegebenenfalls einfach oder mehrfach unterbrochen durch Heteroatome ausgewählt aus O, S, N oder **-N$R^{13}$-,**
wobei

niemals zwei Heteroatome direkt benachbart sind,
und
$R^{13}$ einen linearen, cyclischen oder verzweigten, substituierten oder unsubstituierten Alkylrest mit 1 bis 18 Kohlenstoffatomen oder einen Arylrest mit 6 bis 18 Kohlenstoffatomen bedeutet.

Nicht abschließende Beispiele für Reste $\mathbf{R^5}$ in der Verbindung der allgemeinen Formel V mit **o = 1** sind Alkylreste wie der Methyl-, Ethyl-, n-Propyl-, iso-Propyl-, n-Butyl-, 2-Butyl-, iso-Butyl-, tert.-Butyl-, n-Pentyl-, iso-Pentyl-, neo-Pentyl-, tert.-Pentylrest, Hexylreste wie der n-Hexylrest, Heptylreste wie der n-Heptylrest, Octylreste wie der n-Octylrest und iso-Octylreste wie der 2,2,4-Trimethylpentylrest, Nonylreste wie der n-Nonylrest, Decylreste wie der n-Decylrest, der n-Tetradecyl, der n-Hexadecylrest, der n-Octadecylrest, Cycloalkylreste wie Cyclopentyl-, Cyclohexyl-, 4-Ethylcyclohexyl-, Cycloheptylreste, Norbornylreste und Methylcyclohexylreste oder auch Alkenylreste wie der 2-Propen-2-yl-, Allyl-, 3-Buten-1-yl-, 5-Hexen-1-yl-, 10-Undecen-1-yl-, Octadec-Z-9-enyl-Rest, Cycloalkenylreste wie 2-Cyclohexenyl-, 3-Cyclohexenyl-, Cyclopentadienylrest, 2-(Cyclohex-3-en-1-yl)ethyl, Arylreste wie der Phenyl-, Biphenylyl-, Naphthylrest, Alkarylreste wie o-, m-, p-Tolylreste und Phenethylreste wie 2-Phenylethyl-, 1-Phenylethylreste, E-2-Phenyl-ethen-1-ylreste und Aralkylreste wie der Benzylrest, substituierte Reste wie Halogenalkyl-Reste wie der Chlorethyl-, der Chlorpropyl- und der 3,3,3-Trifluorpropylrest, Alkoxyalkylreste wie der 1-n-Butoxymethyl-, 2-Methoxyethyl-, Tetrahydro-2-furanmethyl, 2-Furanmethyl, Epoxyalkylreste wie der Epoxy-methyl, 1,2-Epoxyethyl-, 1,2-Epoxy-3-propyl- oder 1,2-Epoxy-4-butylrest, Polyethylenglykolalkylreste wie der 2-ω-Methylpolyethylenglglykol-ethyl-Rest, Aminoalkylreste wie der N,N-Dimethylaminoethyl- und der N,N-Dimethylaminopropylrest, quaternäre Ammoniumreste wie der 2-Trimethylammoniumchlorid-1-ethylrest.

Nicht abschließende Beispiele für Reste $\mathbf{R^5}$ in der Verbindung der allgemeinen Formel V mit **o = 2** sind Alkylenreste wie der 1,2-Ethylen-, der 1,2-Propylen, der 1,3-Propylen-, der 1,4-Butylenrest, Arylenreste wie der 1,2-Phenylenrest, Ethlyenglykolreste, Propylenglykolreste, Butylenglykolreste, Polyethylenglykolreste, Polypropylenglykolreste oder Polybutylenglykolreste oder Aminoalkylenreste wie der N-Methylamino-bis-ethylenrest.

Nicht abschließende Beispiele für Verbindungen der allgemeinen Formel V mit **o = 1** sind Methanol, Ethanol, n-Propanol, 2-Propanol, 1-n-Butanol, Natrium-n-butanolat, Isobutanol, 2-Butanol, t-Butanol, Kalium-tert.-butanolat, Natrium-tert.-butanolat, 1-n-Pentanol, 3-Methylbutan-1-ol, 3-Pentanol, 1-n-Octanol, 2-Ethylhexan-1-ol, 1-n-Dodecanol, 1-n-Hexadecanol, 2,2-Dimethyl-1-propanol, 1-n-Butoxymethanol, 3-Methoxy-1-propanol, 3-Methoxy-1-butanol, Tetrahydro-2H-pyran-2-methanol, 2-Chlorethanol, 3-Chlorpropan-1-ol, 3,3,3-Trifluorpropanol, 2-Bromethanol, 2-Iodethanol, 3-Iodpropan-1-ol, 3-Brom-1-propanol, 4-Chlorbutanol, Glycidylether, Glycerincarbonat, 2-Glycidyloxyethanol Ethanolamin-Hydrochlorid, N,N-Dimethylaminoethanol, Propargylalkohol, 2-Butin-1-ol, 3-Butin-1-ol, 3-Butin-2-ol, Allylalkohol, 2-Allyloxyethanol, 2-Methyl-2-propen-1-ol, E-Crotylalkohol, Z-Crotylalkohol, 3-Buten-1-ol, 3-Buten-2-ol, 10-Undecen-1-ol, Oleylalkohol, Poly(ethylenglykol)mono-methylether verschiedener Molmassen wie mPEG6-OH, mPEG7-OH, mPEG12-OH, Poly(ethylenglykol)mono-allylether verschiedener Molmassen Poly(propylenglykol)mono-methylether verschiedener Molmassen, Tergitol® (mono-Butyl-PEG/PPG-Copolymer), Hydroxypropionitril, DL-Lactonitril, Hydroxyaceton, 2-Hydroxyethylacrylat, 3-Hydroxypropylacrylat, 4-Hydroxybutylacrylat, 2-Hydroxyethylmethacrylat, 3-Hydroxypropylmethacrylat, 4-Hydroxybutylmethacrylat, Triethylcitrat, Fettsäurediglyceride Dodecylamin, Oleylamin, Acetamid, N-Methylacetamid, Laurylamid, Oleylamid, Erucasäurelaurylamid, Maleimid, Succinimid, N-Hydroxysuccinimid, N-2-Hydroxyethylsuccinimid, 2-Phenylethanol, Zimtalkohol, Methylsalicylat, Phenol, 3-Methoxyphenol, 2-Methoxyphenol, 3-Chlorphenol, Anilin, N-Methylanilin, Morpholin, Pyrrolidin, Imidazolin, Piperidin, Cystein oder Cholin.

Nicht abschließende Beispiele für Verbindungen der allgemeinen Formel V mit **o = 2** sind 1,2-Ethandiol, 1,2-Propandiol, 1,3-Propandiol, 1,4-Butandiol, 1,2-Butandiol, 1,5-Pentandiol, 2-Methyl-1,3-propandiol, 2,2-Dimethyl-1,3-propandiol, 1,6-Hexandiol, 1,9-Nonandiol, 2-Butin-1,4-diol, Z-2-Buten-1,4-diol, 3-Methyl-2-buten-1-ol Ethanolamin, O-(2-Aminoethanol)polyethylenglykol, 3-Amino-1-propanol, 4-Amino-1-butanol, 2-(Methylamino)ethanol, Amino-2-propanol, DL-Alaninol, Tetrahydro-2,5-furandimethanol, Diethylenglykol, Triethylenglykol, Polyethylenglykol-bisaminopropylamin, Lysin, Fettsäuremonoglyceride wie Glycerinmonostearat Bisphenol-A, 4,4'-(1-Methylethyliden)bis-phenol, Dihydroxyaceton, Ethylendiamin, 1,4-Diaminobutan, 1,6-Diaminohexan, N,N'-Dimethylethylendiamin, Imidazolidin, Piperazin, Polyoxyalkylenamine (Jeffamine®), Jeffamine® T403, Triethanolamin, N,N'-Dimethylharnstoff, 1,3-Bis(hydroxymethyl)harnstoff 2,2'-Thiodiethanol.

Nicht abschließende Beispiele für Verbindungen der allgemeinen Formel V mit **o > 3** sind Glycerin, Pentaerythrit, Di-Pentaerythrit, Sorbit, Mannit, Isomalt, Maltit, Lactit, Xylit, Erythrit, 1,1,1-Tristrimethylolpropan, 1,1,1-Tris-trimethylolethan Saccharide: Glucose, Saccharose, Galaktose, Lactose, Maltose, Diethanolamin, Polyethylenimin oder 1,3-Diamino-2-propanol.

Zur Umsetzung von Verbindungen der allgemeinen Formel V mit dem Poly-3-hydroxy-alkanoat-Carbonsäurechlorid der allgemeinen Formel IV wird dieses vorzugsweise vorgelegt und die Verbindung der allgemeinen Formel V zudosiert. Dies hat den Vorteil, dass kein Umfüllvorgang notwendig ist.

Für den vollständigen Umsatz aller X-Y-Funktionen in der Verbindung der allgemeinen Formel V wird vorzugsweise ein äquimolares Verhältnis der Cl-Funktionalität in dem Poly-3-hydroxy-alkanoat-Carbonsäurechlorid der allgemeinen Formel IV zu der X-Y-Funktionalität in der Verbindung der allgemeinen Formel V gewählt. Handelt es sich dabei um monofunktionelle (**o = 1**) und leicht abtrennbare Verbindungen der allgemeinen Formel V, können diese vorzugsweise zur Beschleunigung der Umsetzung in einem molaren Überschuss bezüglich des Poly-3-hydroxy-alkanoat-Carbonsäurechlorids der allgemeinen Formel IV eingesetzt werden, vorzugsweise im 1,1 bis 10-fachen molaren Überschuss, besonders bevorzugt im 1,1 bis 2-fachen molaren Überschuss. Nicht umgesetzte Anteile können bei Bedarf dann nach beendeter

Reaktion beispielsweise destillativ oder durch Phasentrennung abgetrennt werden.

Soll die Umsetzung der X-Y-Gruppen in der Verbindung der allgemeinen Formel V mit **o > 1** nur unvollständig erfolgen, so ist vorzugsweise ein Überschuss an der Verbindung der allgemeinen Formel V einzusetzen. Das optimale Molverhältnis von der Verbindung der allgemeinen Formel V zu dem Poly-3-hydroxy-alkanoat-Carbonsäurechlorid der allgemeinen Formel IV kann leicht in Vorversuchen ermittelt werden. Es richtet sich nach dem X-Y-Gehalt in der Verbindung der allgemeinen Formel V und dem gewünschten Umsetzungsgrad der vorhandenen X-Y-Gruppen. Eine kinetische Kontrolle kann darüber hinaus über die Dosierung erfolgen, indem die Verbindung der allgemeinen Formel V vorzugsweise im Überschuss vorgelegt und das Poly-3-hydroxy-alkanoat-Carbonsäurechlorid der allgemeinen Formel IV zudosiert wird.

Die Umsetzung erfolgt vorzugsweise im Temperaturbereich zwischen 0°C und 150°C, bevorzugt zwischen 20°C und 100°C, besonders bevorzugt zwischen 50°C und 90°C unter dem Druck der umgebenden Atmosphäre, wobei auch höhere oder niedrigere Drücke eingestellt werden können. Sofern es sich bei Y-Cl um eine flüchtige Verbindung handelt, kann es vorteilhaft sein, zur Beschleunigung der Y-Cl-Entfernung aus der Reaktionsmischung den Druck zu reduzieren, vorzugsweise auf den Bereich von 10 hPa bis 900 hPa, besonders bevorzugt zwischen 100 hPa und 600 hPa, insbesondere zwischen 100 hPa und 300 hPa. Unterstützend kann Inertgas durch die Reaktionsmischung geleitet werden gegebenenfalls in Kombination mit reduziertem Druck.

Die Bedingungen werden vorzugsweise so gewählt, dass die Komponenten der Reaktionsmischung während der Umsetzung nicht abdestilliert werden.

Für den Fall, dass **Y** ein Wasserstoffatom bedeutet, werden zur Entfernung des freigesetzten Chlorwasserstoffs insbesondere bei Einsatz von Aminen vorzugsweise Hilfsbasen wie Ammoniak, tertiäre Amine wie Triethylamin, oder Tri-n-butylamin oder, sofern basischer Stickstoff in der Verbindung der allgemeinen Formel V vorhanden ist, ein entsprechender Überschuss an Verbindung der allgemeinen Formel V oder basische Salze wie Alkalioxide, -hydroxide, -carbonate, -amide, -hydride, oder Erdalkalioxide, -hydroxide, -carbonate, -amide, -hydride verwendet. Die Einsatzmenge an Hilfsbase richtet sich nach der freigesetzten HCl-Menge. Vorzugsweise werden 1 - 5 Mol, besonders bevorzugt 1,05 - 2 Mol, insbesondere 1,05 - 1,2 Mol Base, gerechnet als Basenäquivalent, pro Mol HCl eingesetzt. Die Abtrennung der Neutralisationsprodukte erfolgt vorzugsweise bei der Aufarbeitung der Poly-3-hydroxy-alkanoate der allgemeinen Formel VI durch Filtration, Sedimentation, Zentrifugieren oder Waschen gemeinsam mit dem Katalysator oder dem deaktivierten Katalysator.

Das Verfahren ist dadurch gekennzeichnet, dass Schritt 3 bevorzugt lösungsmittelfrei durchgeführt wird. Etwaige Verunreinigungen der Edukte oder Zwischenprodukte durch Lösungsmittelreste können enthalten sein.

Ein Lösungsmittel kann beispielsweise zur Verbesserung der Durchmischung zugegeben werden. Bevorzugte Lösungsmittel besitzen gute Lösungseigenschaften, verhalten sich gegenüber den Bestandteilen der Reaktionsmischung inert, lassen sich nachträglich leicht abtrennen, beispielsweise für ein Recycling, sind kommerziell verfügbar und gesundheitlich sowie sicherheitstechnisch möglichst unbedenklich.

Nicht abschließende Beispiele dafür sind Anisol, Xylol, Alkane wie n-Heptan, n-Octan, n-Decan, Isoalkane wie Isopar® E von Exxon, Ether wie Di-n-butylether, 2-Methyl-tetrahydrofuran, Methyl-t-butylether, Essigsäuremethylester, Essigsäureethylester, Essigsäurebutylester oder Gemische davon. Besonders bevorzugt ist Anisol. Andere mögliche Lösungsmittel sind Toluol, Tetrahydrofuran, Chlorbenzol, N,N-Dimethylformamid, Dimethylsulfoxid, Dichlormethan oder Trichlormethan.

Das freigesetzte HCl-Gas kann ggf. nach entsprechender Aufbereitung, z.B. in einem Wäscher, einem Recycling zugeführt werden. Ebenso können ggf. anfallende Amin-Hydrochloride einem Recycling zugeführt werden.

Die mittleren Molmassen $\mathbf{M_w}$ der Poly-3-hydroxy-alkanoate der allgemeinen Formel VI aus Schritt 3 des erfindungsgemäßen Verfahrens liegen vorzugsweise im Bereich der mittleren Molmassen $\mathbf{M_w}$ der Poly-3-hydroxy-alkanoate der allgemeinen Formel III beziehungsweise der Poly-3-hydroxyalkanoat-Carbonsäurechloride der allgemeinen Formel IV.

Die mittleren Molmassen $\mathbf{M_w}$ können beispielsweise über NMR-Spektroskopie, SEC, GPC, LC, MALDI-TOF oder ESI-MS bestimmt werden.

Ein weiterer Gegenstand der vorliegenden Erfindung ist die Verwendung der Poly-3-hydroxy-alkanoat-Carbonsäurechloride der allgemeinen Formel IV zur chemischen Modifizierung von pharmazeutischen Wirkstoffen.

Ein weiterer Gegenstand der vorliegenden Erfindung ist die Verwendung der Poly-3-hydroxy-alkanoate der allgemeinen Formel VI in kosmetischen Zusammensetzungen wie beispielsweise Körper- oder Haarpflegeprodukten, Pestiziden, Klebstoffen, in der Textil- oder Lederbehandlung/ -pflege oder als Weichmacher.

Das erfindungsgemäße Verfahren hat den Vorteil, dass die Reaktionsschritte sehr einfach und effizient nacheinander durchgeführt werden können und dabei ohne aufwändige Aufarbeitungsschritte zielgerichtet wie atomökonomisch die Herstellung der gewünschten Poly-3-hydroxy-alkanoate-Carbonsäurechloride der allgemeinen Formel IV und die gewünschten Poly-3-hydroxy-alkanoate der allgemeinen Formel VI zugänglich sind.

Neben den wirtschaftlichen Vorteilen ermöglicht das erfindungsgemäße Verfahren durch seine breite Toleranz funktioneller Gruppen die Synthese einer Vielzahl von Poly-3-hydoxy-alkanoat-Carbonsäurechlorid-Strukturen der allgemeinen Formel IV sowie Poly-3-hydroxy-alkanoat-Strukturen der allgemeinen Formel VI, die beispielweise für die Modifizierung von Pharmaka oder auch als biologisch abbaubare Silikon-Ersatzstoffe in für Silikon-typischen Anwendungen eingesetzt werden können. Besonders vorteilhaft an der erfindungsgemäßen Reaktionsführung ist, dass für die Synthese der Poly-3-hydroxy-alkanoate der allgemeinen Formel III technische Qualitäten der β-Lactone der allgemeinen Formel I direkt eingesetzt werden können.

Ein weiterer Vorteil ist außerdem die kurze Reaktionszeit von wenigen Minuten bis wenigen Stunden verbunden mit einer hohen Selektivität bei gleichzeitig hohem Umsatz. Das nichtumgesetzte Reaktionsprodukt kann einfach abdestilliert und wiederverwendet werden. Der heterogene Katalysator ist in der Regel kommerziell verfügbar, kann ohne Vorbehandlung verwendet und problemlos abgetrennt werden. Ein Lösungsmittel ist nicht notwendig.

Dadurch leistet das erfindungsgemäße Verfahren einen wesentlichen Beitrag zur ökonomischen und ökologischen Nachhaltigkeit.

Der Gegenstand der vorliegenden Erfindung soll durch die nachfolgenden Beispiele erläutert werden, ohne jedoch diese auf die darin offenbarten Inhalte zu beschränken.

Sofern nicht anders angegeben, werden die folgenden Beispiele bei einem Druck der umgebenden Atmosphäre, also bei etwa 1000 hPa, und bei Raumtemperatur, also etwa 20°C bzw. einer Temperatur, die sich beim Zusammengeben der Reaktanden bei Raumtemperatur ohne zusätzliche Heizung oder Kühlung einstellt, durchgeführt.

**Beispiel 1: Herstellung eines PHB-Propylenglykol-Derivates**

**Schritt 1:** Ringöffnungspolymerisation

In einem 1l-3-Hals-Kolben mit Destillationsaufsatz werden 600 g β-Butyrolacton (Quelle: Sigma-Aldrich, enthält laut $^1$H-NMR-Spektrum 1% Crotonsäure) mit 12 g Kaliumcarbonat (wasserfrei, Merck) versetzt. Die Suspension wird im Ölbad bei 95-99°C 1,5 Stunden gerührt. An einer entnommenen Probe wird mittels $^1$H-NMR der Umsatz bestimmt: Der Umsatz liegt bei 83% des eingesetzten β-Butyrolactons. Bezogen auf die entstandene Poly-3-hydroxy-butyrat-Carbonsäure haben sich 3% Crotonsäure gebildet.

Die Mischung wird bei vermindertem Druck (150 hPa) bis 160°C entflüchtigt. Dabei wird nicht umgesetztes β-Butyrolacton abdestilliert. Es kann für weitere Umsetzungen eingesetzt werden. Vom Rückstand wird eine Probe entnommen und filtriert. Aus ihrem $^1$H-NMR-Spektrum ergibt sich folgende mittlere Zusammensetzung der gebildeten Poly-3-hydroxy-butyrat-Carbonsäure:

$$(H_3C\text{-}CH{=}CH\text{-}C(O)\text{-}O\text{-}[CH(CH)_3\text{-}CH_2\text{-}C(O)O]_{8,5}CH(CH_3)CH_2\text{-}C(O)OH)$$

**Schritt 2:** Chlorierung

Die Reaktionsmischung aus Schritt 1 wird bei 40°C mit 190 g Thionylchlorid (Merck) versetzt und 1,5 Stunden bei 40°C gerührt. Der Umsatz kann dabei über die Gasentwicklung leicht verfolgt werden. Nach beendeter Reaktion wird bei 50°C und vermindertem Druck entflüchtigt. Vom Rückstand wird eine Probe entnommen und filtriert. Aus ihrem $^1$H-NMR-Spektrum ergibt sich folgende mittlere Formel des gebildeten Poly-3-hydroxy-butyrat-Carbonsäurechlorids bei einem Umsatz von 99%:

$$(H_3C\text{-}CH{=}CH\text{-}C(O)\text{-}O\text{-}[CH(CH)_3\text{-}CH_2\text{-}C(O)O]_{7,8}CH(CH_3)CH_2\text{-}C(O)Cl)$$

**Schritt 3:** Derivatisierung mit 1,2-Propandiol

Die Reaktionsmischung aus Schritt 2 wird mit 200 g Dichlormethan zur Verbesserung der Rührbarkeit versetzt, anschließend werden bei 40°C 122 g 1,2-Propandiol binnen 20 Minuten zugetropft. Man rührt die Mischung 2 Stunden bei 40°C und legt anschließend für eine weitere Stunde bei 40°C 100 hPa Wasserstrahlvakuum an. Abschließend wird bis 100°C und 1 hPa ausgeheizt. Der Rückstand wird filtriert. Es werden 310 g eines klaren, bräunlichen Filtrats erhalten, dessen $^1$H-NMR-Spektrum eine vollständige Umwandlung der Carbonsäurechlorid-Gruppen in Hydroxyalkoxygruppen anzeigt. Dem derivatisierten Poly-3-hydroxy-butyrat kann aus dem $^1$H-NMR-Spektrum die folgende mittlere Formel zugeordnet werden:

60% 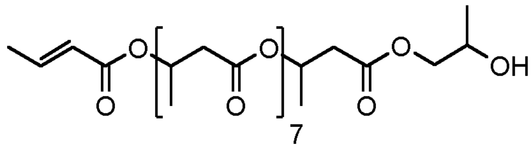

40% 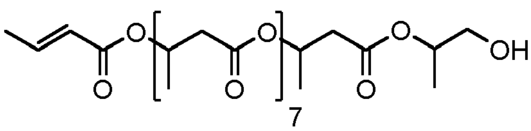

Schritt 1 und 2 in Beispiel 1 werden wiederholt. Die Zusammensetzung des Poly-3-hydroxy-butyrat-Carbonsäurechlorids wird mittels $^1H$-NMR-Spektrum an einer filtrierten Probe bestimmt. Es hat demnach folgende mittlere Zusammensetzung:

$$(H_3C\text{-}CH\text{=}CH\text{-}C(O)\text{-}O\text{-}[CH(CH)_3\text{-}CH_2\text{-}C(O)O]_{8,5}CH(CH_3)CH_2\text{-}C(O)Cl)$$

Die erhaltene Menge an Poly-3-hydroxy-butyrat-Carbonsäurechlorid wird aufgeteilt und mit verschiedenen Reaktionspartnern wie in den nachfolgenden Beispielen 2 bis 6 beschrieben derivatisiert.

**Beispiel 2: Herstellung eines PHB-Oleylesters**

**Schritt 3:** Derivatisierung mit Oleylalkohol

In einem 100 ml-3-Halskolben werden 30 g der unfiltrierten Reaktionsmischung aus Schritt 2 von Beispiel 1 bei 80°C und schwachem Wasserstrahlvakuum (100 hPa) mit 8,9 g Oleylalkohol (Sigma-Aldrich, 90%ig, Rest: Isomere und andere Fettsäuren im gleichen Molmassenbereich) tropfenweise versetzt. Man rührt eine weitere Stunde bei 80°C, und fügt anschließend 0,2 g Hexamethyldisilazan (Wacker Chemie AG) dazu, um restliche Säure zu neutralisieren sowie gegebenenfalls vorhandene OH-Gruppen zu silylieren. Danach wird bis 120°C/1 hPa eine Stunde lang ausgeheizt. Der braune Rückstand wird bei 50°C mittels Drucknutsche über ein Tiefenfilter Pall T1000 (10-25 μm) filtriert. Man isoliert 38,2 g eines klaren bräunlichen Öls. Gemäß $^1H$-NMR-Spektrum hat das derivatisierte Poly-3-hydroxy-butyrat folgende mittlere Formel:

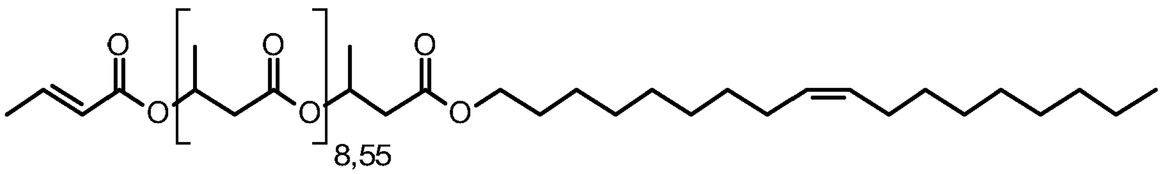

**Beispiel 3: Herstellung eines PHB-2-(N,N-Dimethylamino)ethylesters**

**Schritt 3:** Derivatisierung mit 2-N,N-Dimethylaminoethanol

In einem 100 ml-3-Halskolben werden 20 g der unfiltrierten Reaktionsmischung aus Schritt 2 von Beispiel 1 bei 24°C in 20 g Dichlormethan (Merck, 99,9%) gelöst. Anschließend tropft man unter Rühren eine 50%ige Lösung von 3,9 g 2-N,N-Dimethylaminoethanol (Sigma-Aldrich) in Dichlormethan hinzu. Dabei bildet sich weißer Rauch und die Temperatur der Mischung steigt auf 37°C an. Man rührt weitere 5,5 Stunden bei 25°C, wäscht drei Mal mit je 3,9 g 5%iger wässriger Natriumhydrogencarbonat-Lösung, trocknet die organische Phase über wasserfreiem Magnesiumsulfat, filtriert die Reaktionsmischung über Beco KD3 (2-3,5 μm) und entflüchtigt das klare, braune Filtrat bei 80°C/1 hPa. Man isoliert 14,5 g eines klaren braunen Öls. Gemäß $^1H$-NMR-Spektrum hat das derivatisierte Poly-3-hydroxy-butyrat folgende mittlere Formel:

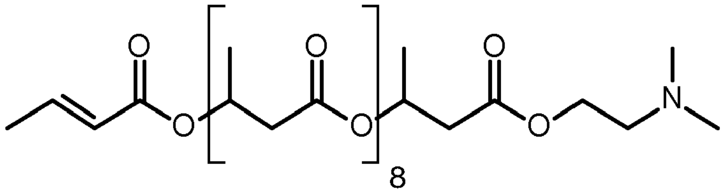

8

**Beispiel 4: Herstellung eines PHB-2-(N,N-Dimethylamino)ethylester-Hydrochlorids**

**Schritt 3:** Derivatisierung mit N,N-Dimethylaminoethanol

In einem 100 ml-3-Halskolben werden 10 g der unfiltrierten Reaktionsmischung aus Schritt 2 von Beispiel 1 bei 24°C in 10 g Anisol (Sigma-Aldrich, zur Synthese) gelöst. Anschließend tropft man unter Rühren 0,9 g N,N-Dimethylaminoethanol (Sigma-Aldrich) hinzu. Man rührt 4 Stunden bei 25°C und heizt eine halbe Stunde bei 50°C/1 hPa. Man isoliert 10,9 g eines viskosen, klaren braunen Öls. Gemäß $^1$H-NMR-Spektrum hat das derivatisierte Poly-3-hydroxybutyrat folgende mittlere Formel:

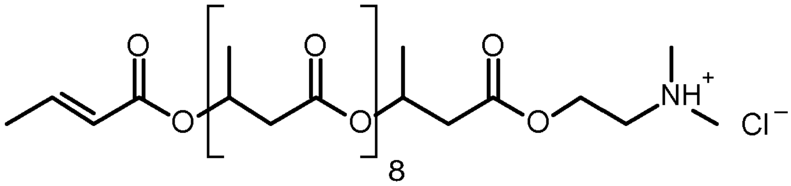

8

**Beispiel 5: Herstellung eines PHB-PEG-350-Me-Esters**

**Schritt 3:** Derivatisierung mit Methoxypolyethylenglykol 350

In einem 100 ml-3-Halskolben werden 30 g der unfiltrierten Reaktionsmischung aus Schritt 2 von Beispiel 1 bei 80°C und schwachem Wasserstrahlvakuum (100 hPa) mit 11,6 g Methoxypolyethylenglykol 350 (mittleres Molgewicht 350 g/mol, Sigma-Aldrich) binnen 20 Minuten tropfenweise versetzt. Man rührt eine weitere Stunde bei 80°C, und fügt anschließend 0,2 g Hexamethyldisilazan (Wacker Chemie AG) dazu, um restliche Säure zu neutralisieren sowie gegebenenfalls vorhandene OH-Gruppen zu silylieren. Danach wird bis 120°C/1 hPa eine Stunde lang ausgeheizt. Der braune Rückstand wird bei 60°C mittels Drucknutsche über ein Tiefenfilter Pall T1000 (10-25 μm) filtriert. Man isoliert 30,1 g eines klaren bräunlichen Öls. Gemäß $^1$H-NMR-Spektrum hat das derivatisierte Poly-3-hydroxy-butyrat folgende mittlere Formel:

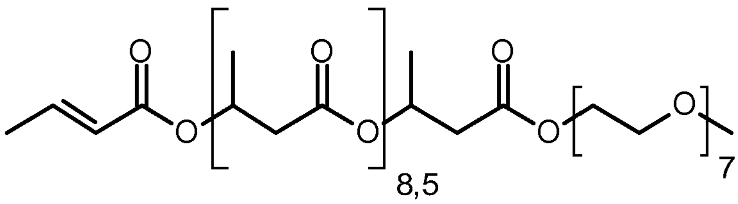

8,5

**Beispiel 6: Herstellung eines PHB-2-Methyl-propan-1-yl-3-ol-Esters**

**Schritt 3:** Derivatisierung mit 2-Methyl-1,3-propandiol

In einem 100 ml-3-Halskolben werden 30 g 2-Methyl-1,3-propandiol (Sigma-Aldrich, 99%) bei 40°C und 100 hPa vorgelegt. Man dosiert 60 g einer filtrierten 50%igen Lösung der Reaktionsmischung aus Schritt 2 in Beispiel 1 in Anisol (Sigma-Aldrich, zur Synthese) innerhalb 50 Minuten zu, lässt weitere 5 Stunden bei 40°C und 80-100 hPa rühren und heizt anschließend bis 140°C/0,1 hPa eine Stunde lang aus. Man isoliert 30,1 g eines klaren bräunlichen Öls. Gemäß $^1$H-NMR-Spektrum hat das derivatisierte Poly-3-hydroxy-butyrat folgende mittlere Formel:

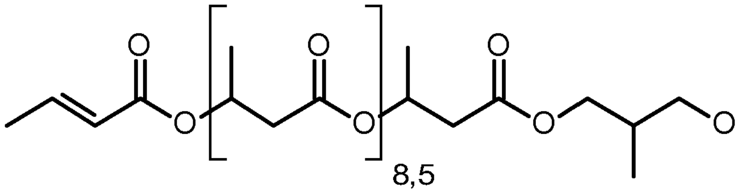

**Beispiel 7: Herstellung eines PHB-PEG-A-B-A-Blockcopolymers**

**Schritt 1:** Ringöffnungspolymerisation

In einem 100 ml-3-Hals-Kolben mit Destillationsaufsatz werden 60 g β-Butyrolacton (Quelle: Sigma-Aldrich, enthält laut $^1$H-NMR-Spektrum 1% Crotonsäure) mit 1,2 g KF*$Al_2O_3$ versetzt. Die Suspension wird im Ölbad bei 95-99°C zwei Stunden gerührt. An einer entnommenen Probe wird mittels $^1$H-NMR der Umsatz bestimmt: Der Umsatz liegt bei 67% des eingesetzten β-Butyrolactons. Bezogen auf die entstandene Poly-3-hydroxy-butyrat-Carbonsäure haben sich 1,5% Crotonsäure gebildet. Die Mischung wird bei vermindertem Druck (150 hPa) bis 160°C entflüchtigt. Dabei wird nicht umgesetztes β-Butyrolacton abdestilliert. Es kann für weitere Umsetzungen eingesetzt werden.

Der Katalysator KF*$Al_2O_3$ wird folgendermaßen hergestellt:

31 g Aluminiumoxid (Merck) werden mit 17,7 g Kaliumfluorid (VWR) unter Stickstoffspülung in einer Reibschale verrieben.

Die Mischung wird im Trockenschrank bei 200°C 24 h erhitzt. Die Lagerung des weißen Pulvers erfolgt unter Stickstoff.

**Schritt 2:** Chlorierung

Die Reaktionsmischung aus Schritt 1 wird bei 40°C mit 28,6 g Thionylchlorid (Merck) versetzt und 2,5 Stunden bei 40°C gerührt. Der Umsatz kann dabei über die Gasentwicklung leicht verfolgt werden. Nach beendeter Reaktion wird bei 50°C und vermindertem Druck entflüchtigt. Als Rückstand verbleibt ein viskoses Öl, das nach $^1$H-NMR folgende Zusammensetzung aufweist:

$$(H_3C\text{-}CH{=}CH\text{-}C(O)\text{-}O\text{-}[CH(CH)_3\text{-}CH_2\text{-}C(O)O]_{7,44}CH(CH_3)CH_2\text{-}C(O)\text{-}Cl)$$

**Schritt 3:** Derivatisierung mit PEG400

4,5 g Poly-3-hydroxy-butyrat-Carbonsäurechlorid aus Schritt 2 werden bei 80°C/200 hPa mit 1 g Polyethylenglykol 400 (Fluka) tropfenweise versetzt. Man rührt unter diesen Bedingungen noch eine weitere Stunde, bricht das Vakuum mit Stickstoff und gibt zum Abfangen von Säureresten 0,02 g Hexamethyldisilazan (Wacker Chemie AG) zu und rührt weitere 5 Minuten. Anschließend wird bis 120°C/1 hPa entflüchtigt. Der Rückstand wird filtriert. Man isoliert 5 g eines klaren orangefarbenen Öls, welches laut $^1$H-NMR-Spektrum folgende Zusammensetzung aufweist:

$$(H_3C\text{-}CH{=}CH\text{-}C(O)O[CH(CH)_3\text{-}CH_2\text{-}C(O)O]_{8,4}CH(CH_2CH_2O)_{8,7}[(O)CCH_2\text{-}CH(CH_3)\text{-}O]_{8,4}\text{-}C(O)\text{-}CH{=}CH\text{-}CH_3)$$

**Beispiel 8: Herstellung eines PHB-4-Acryloxybutylesters**

**Schritt 1:** Ringöffnungspolymerisation

Beispiel 1 wird wiederholt, jedoch wird bei Schritt 1 die Reaktion nach 60% Umsatz durch Abdestillieren des nicht umgesetzten β-Butyrolactons bei 100°C/1,5 hPa abgebrochen. Bezogen auf die entstandene Poly-3-hydroxy-butyrat-Carbonsäure haben sich 1,7% Crotonsäure gebildet. Das als Zwischenprodukt gebildete Poly-3-hydroxy-butyrat besitzt laut $^1$H-NMR-Spektrum einer entnommenen Probe folgende mittlere Zusammensetzung:

$$(H_3C\text{-}CH{=}CH\text{-}C\ (O)\text{-}O\text{-}[CH(CH)_3\text{-}CH_2\text{-}C(O)O]_{13,5}CH(CH_3)CH_2\text{-}C(O)OH)$$

**Schritt 2:** Chlorierung

Für die Chlorierung wird der Rückstand bei 40°C mit der 5-fachen molaren Menge bezüglich Carboxygruppen der Poly-3-hydroxy-butyrat-Carbonsäure an Thionylchlorid versetzt. Man lässt bei 2 h bei 40°C reagieren und zieht bei 0,5 hPa bis 50°C flüchtige Bestandteile ab. Laut $^1$H-NMR-Spektrum besitzt der hellgelbe viskose Rückstand folgende Zusammensetzung:

$$(H_3C\text{-}CH\text{=}CH\text{-}C\ (O)\text{-}O\text{-}[CH(CH)_3\text{-}CH_2\text{-}C(O)O]_{12,5}CH(CH_3)CH_2\text{-}C(O)Cl)$$

**Schritt 3:** Derivatisierung mit 4-Hydroxybutylacrylat

In einem 250 ml-3-Halskolben werden 39,6 g der unfiltrierten Reaktionsmischung aus Schritt 2 bei 40°C und schwachem Wasserstrahlvakuum (100 hPa) mit 7,6 g 4-Hydroxybutylacrylat (TCI, 90%ig) tropfenweise versetzt. Man rührt eine weitere Stunde bei 40°C. Danach wird bis 100°C/0,4 hPa 15 Minuten lang ausgeheizt. Der hellbraune viskose Rückstand wird bei 40°C mittels Drucknutsche über ein Tiefenfilter Pall T1000 (10-25 μm) filtriert. Man isoliert 42 g eines klaren bräunlichen Öls. Gemäß $^1$H-NMR-Spektrum hat das derivatisierte Poly-3-hydroxy-butyrat folgende mittlere Formel:

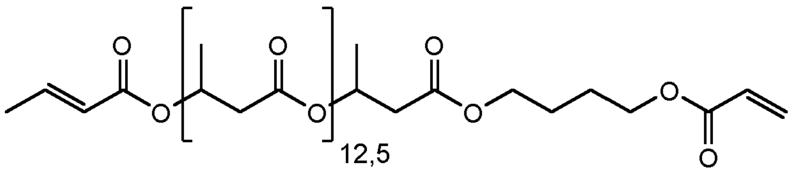

**UV-Vernetzung:**

2 g des acrylfunktionellen Poly-3-hydroxy-butyrats werden mit Photoinitiator Darocur® 1173 (Sigma-Aldrich, 2-Methyl-1-Phenyl-Propan-2-ol-1-one) vermischt. Ein Teil der klaren Mischung wird in einer Schichtdicke von ca. 0,1 mm in eine Glaswanne gegossen und durch UV-Bestrahlung in einer UV-Kammer (UVACUBE, Hönle, Hg-Halogenid-Lampe, 290-415 nm, 2000W) innerhalb von 15 Sekunden zu einem klaren, elastischen Vulkanisat vernetzt.

**Vergleichsbeispiel 1: Veresterung von Poly-3-hydroxy-butyrat-Carbonsäure mit PEG400 gemäß Stand der Technik**

Am Wasserabscheider werden 25 g einer Poly-3-hydroxy-butyrat-Carbonsäure mit einer Crotonsäureester-Endgruppe und einer Carbonsäureendgruppe der mittleren Formel ($H_3C\text{-}CH\text{=}CH\text{-}C(O)\text{-}O\text{-}[CH(CH)_3\text{-}CH_2\text{-}C(O)O]_{8,67}H$) gemäß Schritt 1 aus Beispiel 1 (entflüchtigt und filtriert) hergestellt und mit 5,9 g Polyethylenglykol 400 (Fluka), 10 g Anisol (Merck) und 0,1 g Titantetraisopropylat (Merck) bis zur Beendigung der Wasserentwicklung (5,5 Stunden) unter Rückfluss gekocht.

Es bilden sich dabei massive, kristalline Beläge aus Crotonsäure im Dampfraum, da sich das Polymer zersetzt.

Laut $^1$H-NMR-Spektrum des Rückstands liegt der Umsatz der Carbonsäurefunktion zur Esterfunktion bei 60%. Die Anzahl der Estereinheiten hat sich von 8,67 auf 3,6 verringert, was den Abbau zu Crotonsäure bestätigt.

Unter diesen für eine Veresterung typischen Reaktionsbedingungen wird die Polyesterkette massiv abgebaut.

## Patentansprüche

1. Verfahren zur Herstellung von Poly-3-hydroxy-alkanoaten der allgemeinen Formel VI, welche an dem einen Kettenende eine Ester-Einheit als funktionelle Gruppe und an dem anderen Kettenende eine weitere funktionelle Gruppe aufweisen, umfassend die folgenden Schritte:

   **Schritt 1: Ringöffnungspolymerisation,** bei der mindestens ein β-Lacton der allgemeinen Formel I mit mindestens einer ungesättigten Carbonsäure der allgemeinen Formel II in Anwesenheit mindestens eines heterogenen Katalysators zu dem Poly-3-hydroxy-alkanoat der allgemeinen Formel III umgesetzt wird,

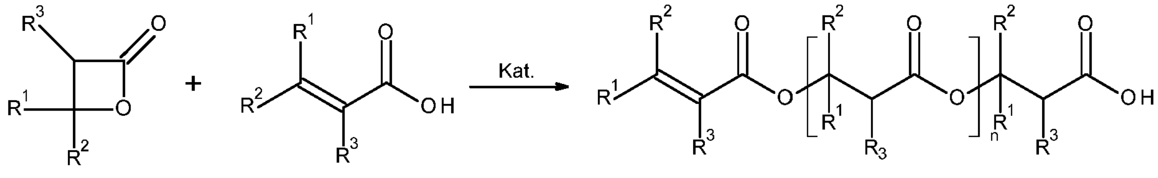


I II III

**Schritt 2**: **Chlorierung,** bei der das Poly-3-hydroxy-alkanoat der allgemeinen Formel III mit mindestens einem Chlorierungsmittel Cl umgesetzt wird, so dass das Poly-3-hydroxy-alkanoat-Carbonsäurechlorid der allgemeinen Formel IV entsteht

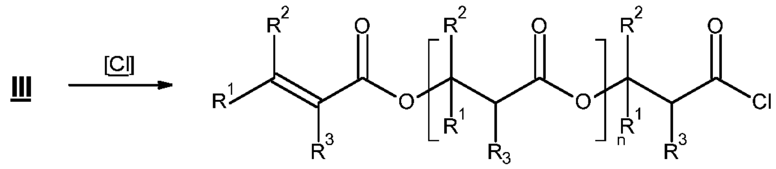


IV

und

**Schritt 3: Derivatisierung,** bei der das Poly-3-hydroxy-alkanoat-Carbonsäurechlorid der allgemeinen Formel IV mit mindestens einer mindestens eine funktionelle Gruppe X-Y aufweisenden Verbindung der allgemeinen Formel V zu dem Poly-3-hydroxy-alkanoat der allgemeinen Formel VI unter Abspaltung von Y-Cl umgesetzt wird,

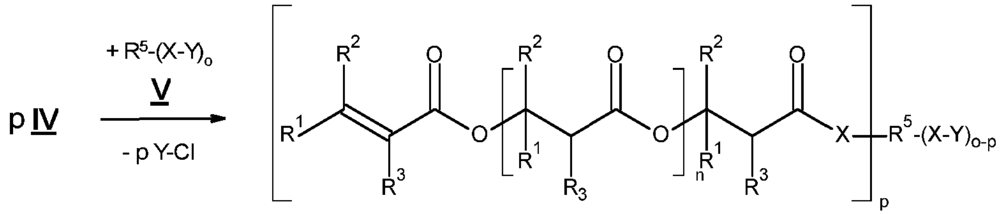


VI

wobei

**$R^1$, $R^2$, $R^3$** unabhängig voneinander ein Wasserstoffatom, ein Halogenatom, einen linearen, (bi)cyclischen oder verzweigten, substituierten oder unsubstituierten $C_1$ - $C_{18}$ Kohlenwasserstoffrest, gegebenenfalls unterbrochen durch Heteroatome ausgewählt aus O, S oder N, bedeuten, wobei die Reste **$R^1$, $R^2$, $R^3$** auch paarweise verbunden als Bestandteil cyclischer Strukturen vorliegen können,

**$R^5$** einen linearen, verzweigten oder (bi)cyclischen, gesättigten oder ungesättigten, monomeren oder polymeren, substituierten oder unsubstituierten Kohlenwasserstoffrest mit 1 - 100 Kohlenstoffatomen bedeutet,

**X** -O- oder -N**$R^{11}$**- bedeutet,

wobei

**$R^{11}$** unabhängig voneinander ein Wasserstoffatom oder einen linearen, cyclischen oder verzweigten, substituierten oder unsubstituierten Alkylrest mit 1 bis 18 Kohlenstoffatomen oder einen Arylrest mit 6 bis 18 Kohlenstoffatomen oder einen Rest der allgemeinen Formel -C(=O)-**$R^{12}$** bedeutet,

wobei

**$R^{12}$** ein Wasserstoffatom oder einen linearen, cyclischen oder verzweigten, substituierten oder unsubstituierten Alkylrest mit 1 bis 18 Kohlenstoffatomen oder einen Arylrest mit 6 bis 18 Kohlenstoffatomen oder einen primären, sekundären oder tertiären Aminrest -N**$R^8R^9$** bedeutet,

wobei

**$R^8$** und **$R^9$** unabhängig voneinander ein Wasserstoffatom oder einen Kohlenwasserstoffrest mit 1

bis 6 Kohlenstoffatomen bedeuten und die beiden Reste **$R^8$** und **$R^9$** paarweise verbunden als Bestandteil cyclischer Strukturen vorliegen können,

**Y** ein Wasserstoffatom, einen Metallrest oder einen metallhaltigen Rest bedeutet, wobei das Metall ausgewählt ist aus den Alkalimetallen, den Erdalkalimetallen, Silicium, Titan, Zink, Zinn, Eisen, Mangan oder Kupfer,
**n** 4 bis 1.400 bedeutet,
**o** 1 bis 16 bedeutet
und
**p** ≤ **o** bedeutet.

2. Verfahren nach Anspruch 1, **dadurch gekennzeichnet, dass** als β-Lacton der allgemeinen Formel I ein β-Lacton technischer Qualität mit einer Reinheit von mindestens ≥ 90 Gew.-% eingesetzt wird.

3. Verfahren nach Anspruch 1 oder 2, **dadurch gekennzeichnet, dass** als Initiator für die Ringöffnungspolymerisation mindestens eine ungesättigte Carbonsäure der allgemeinen Formel II fungiert, die einem Isomer des β-Lactons der allgemeinen Formel I entspricht.

4. Verfahren nach Anspruch 1, 2 oder 3, **dadurch gekennzeichnet, dass** als Katalysator ein fester, basischer Katalysator, ausgewählt aus der Gruppe der Alkalimetalloxide, -hydroxide, - carbonate und -hydrogencarbonate oder Alkalimetallfluoride gegebenenfalls auf Trägermaterialien oder Erdalkalimetalloxide- oder -hydroxide eingesetzt wird.

5. Verfahren nach einem der Ansprüche 1 bis 4, **dadurch gekennzeichnet, dass** als Chlorierungsmittel Cl Thionylchlorid, Phosgen oder Phosphor(V)chlorid eingesetzt werden.

6. Verfahren nach einem der Ansprüche 1 bis 5, **dadurch gekennzeichnet, dass** im Batchbetrieb mindestens zwei aufeinanderfolgende Schritte ohne Zwischenaufreinigung im selben Reaktionsgefäß oder alle Schritte in einem semi- oder vollkontinuierlichen Verfahren in nacheinander geschalteten Reaktoren hergestellt werden.

7. Verfahren nach einem der Ansprüche 1 bis 6, **dadurch gekennzeichnet, dass $R^5$** einen linearen, verzweigten oder (bi)cyclischen, gesättigten oder ungesättigten, monomeren oder polymeren, substituierten oder unsubstituierten Kohlenwasserstoffrest mit 3 - 100 Kohlenstoffatomen bedeutet.

8. Verfahren nach einem der Ansprüche 1 bis 7, **dadurch gekennzeichnet, dass** die funktionelle Gruppe X-Y -OH, $-NHR^{11}$, ein Li-, Na-, K-, Mg- oder Ca-Alkoholat oder ein Sauerstoff-gebundenes Silyl bedeutet.

9. Verfahren nach einem der Ansprüche 1 bis 8, **dadurch gekennzeichnet, dass n** 4 bis 100 bedeutet.

10. Verfahren nach einem der Ansprüche 1 bis 9, **dadurch gekennzeichnet, dass** die Schritte 1 bis 3 lösungsmittelfrei durchgeführt werden.

11. Poly-3-hydroxy-alkanoat-Carbonsäurechloride der allgemeinen Formel,

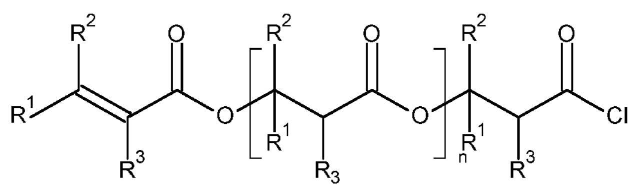

**IV**

wobei

**$R^1$, $R^2$, $R^3$** unabhängig voneinander ein Wasserstoffatom, ein Halogenatom, einen linearen, (bi)cyclischen oder verzweigten, substituierten oder unsubstituierten $C_1$ - $C_{18}$ Kohlenwasserstoffrest, gegebenenfalls unterbrochen durch Heteroatome ausgewählt aus O, S oder N, bedeuten, wobei die Reste **$R^1$, $R^2$, $R^3$** auch paarweise

verbunden als Bestandteil cyclischer Strukturen vorliegen können
und
**n** 4 bis 1.400 bedeutet.

**12.** Poly-3-hydroxy-alkanoate der allgemeinen Formel,

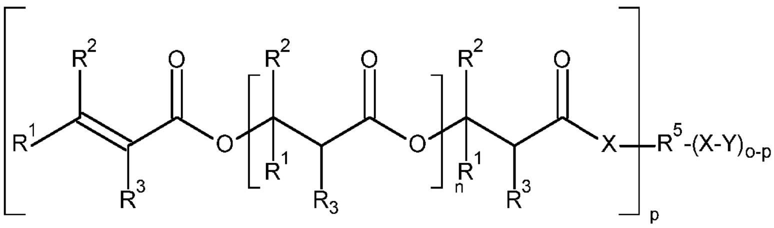

**VI**

wobei

**$R^1$, $R^2$, $R^3$** unabhängig voneinander ein Wasserstoffatom, ein Halogenatom, einen linearen, (bi)cyclischen oder verzweigten, substituierten oder unsubstituierten $C_1$ - $C_{18}$ Kohlenwasserstoffrest, gegebenenfalls unterbrochen durch Heteroatome ausgewählt aus O, S oder N, bedeuten, wobei die Reste **$R^1$, $R^2$, $R^3$** auch paarweise verbunden als Bestandteil cyclischer Strukturen vorliegen können,
**$R^5$** einen linearen, verzweigten oder (bi)cyclischen, gesättigten oder ungesättigten, monomeren oder polymeren, substituierten oder unsubstituierten Kohlenwasserstoffrest mit 1 - 100 Kohlenstoffatomen bedeutet,
**X** -O- oder -N**$R^{11}$**- bedeutet,
wobei
**$R^{11}$** unabhängig voneinander ein Wasserstoffatom oder einen linearen, cyclischen oder verzweigten, substituierten oder unsubstituierten Alkylrest mit 1 bis 18 Kohlenstoffatomen oder einen Arylrest mit 6 bis 18 Kohlenstoffatomen oder einen Rest der allgemeinen Formel -C(=O)-**$R^{12}$** bedeutet,
wobei

> **$R^{12}$** ein Wasserstoffatom oder einen linearen, cyclischen oder verzweigten, substituierten oder unsubstituierten Alkylrest mit 1 bis 18 Kohlenstoffatomen oder einen Arylrest mit 6 bis 18 Kohlenstoffatomen oder einen primären, sekundären oder tertiären Aminrest, -N**$R^8R^9$** bedeutet,
> wobei
> **$R^8$** und **$R^9$** unabhängig voneinander einen Wasserstoffrest oder einen Kohlenwasserstoffrest mit 1 bis 6 Kohlenstoffatomen bedeuten und die beiden Reste **$R^8$** und **$R^9$** paarweise verbunden als Bestandteil cyclischer Strukturen vorliegen können,

**Y** ein Wasserstoffatom, einen Metallrest oder einen metallhaltigen Rest bedeutet, wobei das Metall ausgewählt ist aus den Alkalimetallen, den Erdalkalimetallen, Silicium, Titan, Zink, Zinn, Eisen, Mangan oder Kupfer,
**n** 4 bis 1.400 bedeutet,
**o** 1 bis 16 bedeutet
und
**p** < **o** bedeutet.

**13.** Poly-3-hydroxy-alkanoate der allgemeinen Formel VI nach Anspruch 12, wobei **$R^5$** einen linearen, verzweigten oder (bi)cyclischen, gesättigten oder ungesättigten, monomeren oder polymeren, substituierten oder unsubstituierten Kohlenwasserstoffrest mit 3 - 100 Kohlenstoffatomen bedeutet.

**14.** Poly-3-hydroxy-alkanoate der allgemeinen Formel VI nach Anspruch 12 oder 13, **dadurch gekennzeichnet, dass** die funktionelle Gruppe X-Y -OH, -NHR$^{11}$, ein Li-, Na-, K-, Mg- oder Ca-Alkoholat oder ein Sauerstoff-gebundenes Silyl bedeutet.

**15.** Poly-3-hydroxy-alkanoat-Carbonsäurechloride der allgemeinen Formel IV nach Anspruch 11 oder Poly-3-hydroxy-alkanoate der allgemeinen Formel VI nach Anspruch 12, 13 oder 14, **dadurch gekennzeichnet, dass n** 4 bis 100 bedeutet.

**16.** Verwendung der Poly-3-hydroxy-alkanoat-Carbonsäurechloride der allgemeinen Formel IV gemäß Anspruch 11 oder 15 zur chemischen Modifizierung von pharmazeutischen Wirkstoffen.

**17.** Verwendung der Poly-3-hydroxy-alkanoate der allgemeinen Formel VI gemäß einem der Ansprüche 12 bis 15 in kosmetischen Zusammensetzungen, Pestiziden, Klebstoffen, in der Textil- oder Lederbehandlung/ -pflege oder als Weichmacher.

## Claims

**1.** Process for producing poly-3-hydroxyalkanoates of general formula VI which have an ester unit as functional group at one chain end and a further functional group at the other chain end,
comprising the steps of:

Step 1: Ring-opening polymerization where at least one β-lactone of general formula I is reacted with at least one unsaturated carboxylic acid of general formula II in the presence of at least one heterogeneous catalyst to afford the poly-3-hydroxyalkanoate of general formula III,

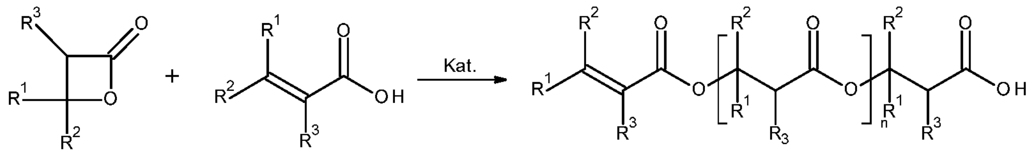


I II III

Step 2: Chlorination where the poly-3-hydroxyalkanoate of general formula III is reacted with at least one chlorinating agent Cl to form the poly-3-hydroxyalkanoate carbonyl chloride of general formula IV

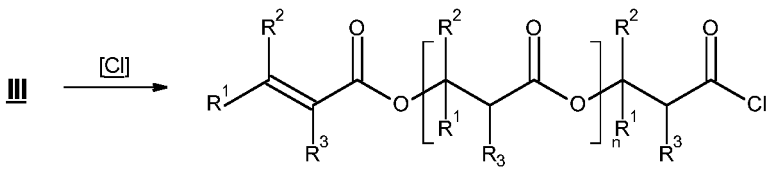


IV

and
Step 3: Derivatization where the poly-3-hydroxyalkanoate carbonyl chloride of general formula IV is reacted with at least one compound of general formula V comprising at least one functional group X-Y to afford the poly-3-hydroxyalkanoate of general formula VI by elimination of Y-Cl,

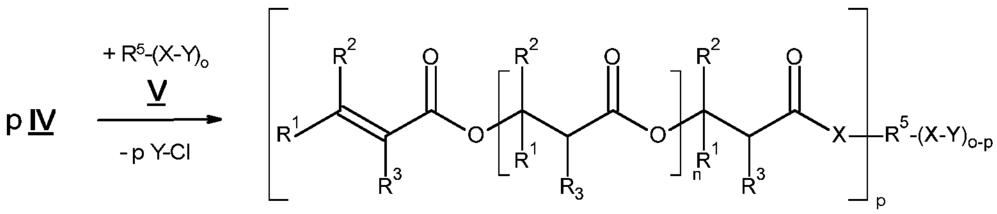


VI

wherein

$R^1$, $R^2$, $R^3$ independently of one another represent a hydrogen atom, a halogen atom, a linear, (bi)cyclic or branched, substituted or unsubstituted $C_1$ - $C_{18}$ hydrocarbon radical, optionally interrupted by heteroatoms selected from O, S or N, wherein the radicals $R^1$, $R^2$, $R^3$ may also be present connected in pairs as a constituent of cyclic structures,
$R^5$ represents a linear, branched or (bi)cyclic, saturated or unsaturated, monomeric or polymeric, substituted

or unsubstituted hydrocarbon radical having 1 - 100 carbon atoms,
X represents -O- or $-NR^{11}-$,
wherein
$R^{11}$ independently at each occurrence represents a hydrogen atom or a linear, cyclic or branched, substituted or unsubstituted alkyl radical having 1 to 18 carbon atoms or an aryl radical having 6 to 18 carbon atoms or a radical of general formula $-C(=O)-R^{12}$,
wherein
$R^{12}$ represents a hydrogen atom or a linear, cyclic or branched, substituted or unsubstituted alkyl radical having 1 to 18 carbon atoms or an aryl radical having 6 to 18 carbon atoms or a primary, secondary or tertiary amine radical-$NR^8R^9$,
wherein
$R^8$ and $R^9$ independently of one another represent a hydrogen atom or a hydrocarbon radical having 1 to 6 carbon atoms and the two radicals $R^8$ and $R^9$ may be present connected in pairs as a constituent of cyclic structures,
Y represents a hydrogen atom, a metal radical or a metal-containing radical, wherein the metal is selected from the alkali metals, the alkaline earth metals, silicon, titanium, zinc, tin, iron, manganese or copper,
n is 4 to 1400,
o is 1 to 16
and

$$p \leq o.$$

**2.** Process according to Claim 1, **characterized in that** the β-lactone of general formula I employed is a β-lactone of technical quality having a purity of at least ≥ 90% by weight.

**3.** Process according to Claim 1 or 2, **characterized in that** it is at least one unsaturated carboxylic acid of general formula II which corresponds to an isomer of the β-lactone of general formula I that functions as the initiator for the ring-opening polymerization.

**4.** Process according to Claim 1, 2 or 3, **characterized in that** the catalyst employed is a solid, basic catalyst selected from the group of alkali metal oxides, hydroxides, carbonates and hydrogencarbonates or alkali metal fluorides optionally on support materials or alkaline earth metal oxides or hydroxides.

**5.** Process according to any of Claims 1 to 4, **characterized in that** the chlorinating agent Cl employed is thionyl chloride, phosgene or phosphorus(V) chloride.

**6.** Process according to any of Claims 1 to 5, **characterized in that** in batch operation at least two successive steps are produced without intermediate purification in the same reaction vessel or in a semi- or fully continuous process all steps are produced in serially arranged reactors.

**7.** Process according to any of Claims 1 to 6, **characterized in that** $R^5$ represents a linear, branched or (bi)cyclic, saturated or unsaturated, monomeric or polymeric, substituted or unsubstituted hydrocarbon radical having 3 - 100 carbon atoms.

**8.** Process according to any of Claims 1 to 7, **characterized in that** the functional group X-Y represents -OH, $-NHR^{11}$, a Li, Na, K, Mg or Ca alkoxide or oxygen-bonded silyl.

**9.** Process according to any of Claims 1 to 8, **characterized in that** n is 4 to 100.

**10.** Process according to any of Claims 1 to 9, **characterized in that** steps 1 to 3 are performed solventlessly.

**11.** Poly-3-hydroxyalkanoate carbonyl chlorides of general formula,

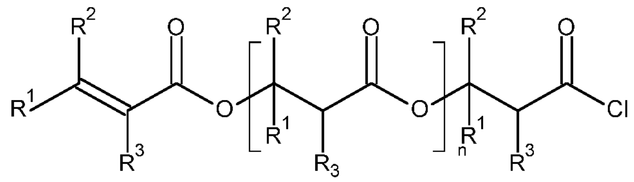

IV

wherein

$R^1$, $R^2$, $R^3$ independently of one another represent a hydrogen atom, a halogen atom, a linear, (bi)cyclic or branched, substituted or unsubstituted $C_1$ - $C_{18}$ hydrocarbon radical, optionally interrupted by heteroatoms selected from O, S or N, wherein the radicals $R^1$, $R^2$, $R^3$ may also be present connected in pairs as a constituent of cyclic structures
and
n is 4 to 1400.

**12.** Poly-3-hydroxyalkanoates of general formula,

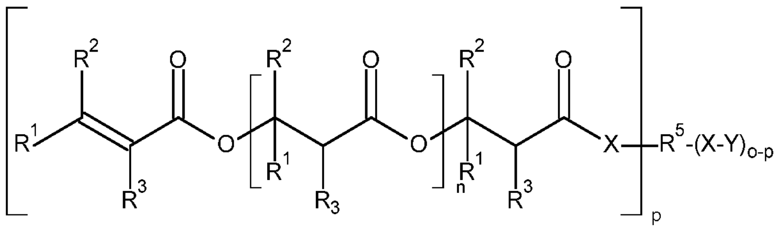

VI

wherein

$R^1$, $R^2$, $R^3$ independently of one another represent a hydrogen atom, a halogen atom, a linear, (bi)cyclic or branched, substituted or unsubstituted $C_1$ - $C_{18}$ hydrocarbon radical, optionally interrupted by heteroatoms selected from O, S or N, wherein the radicals $R^1$, $R^2$, $R^3$ may also be present connected in pairs as a constituent of cyclic structures,
$R^5$ represents a linear, branched or (bi)cyclic, saturated or unsaturated, monomeric or polymeric, substituted or unsubstituted hydrocarbon radical having 1 - 100 carbon atoms,
X represents -O- or $-NR^{11}-$,
wherein
$R^{11}$ independently at each occurrence represents a hydrogen atom or a linear, cyclic or branched, substituted or unsubstituted alkyl radical having 1 to 18 carbon atoms or an aryl radical having 6 to 18 carbon atoms or a radical of general formula $-C(=O)-R^{12}$,
wherein
$R^{12}$ represents a hydrogen atom or a linear, cyclic or branched, substituted or unsubstituted alkyl radical having 1 to 18 carbon atoms or an aryl radical having 6 to 18 carbon atoms or a primary, secondary or tertiary amine radical $-NR^8R^9$ ,
wherein
$R^8$ and $R^9$ independently of one another represent a hydrogen radical or a hydrocarbon radical having 1 to 6 carbon atoms and the two radicals $R^8$ and $R^9$ may be present connected in pairs as a constituent of cyclic structures, Y represents a hydrogen atom, a metal radical or a metal-containing radical, wherein the metal is selected from the alkali metals, the alkaline earth metals, silicon, titanium, zinc, tin, iron, manganese or copper,
n is 4 to 1400,
o is 1 to 16
and

p < o.

**13.** Poly-3-hydroxyalkanoates of general formula VI according to Claim 12, wherein $R^5$ represents a linear, branched or (bi)cyclic, saturated or unsaturated, monomeric or polymeric, substituted or unsubstituted hydrocarbon radical having 3 - 100 carbon atoms.

**14.** Poly-3-hydroxyalkanoates of general formula VI according to Claim 12 or 13, **characterized in that** the functional group X-Y represents -OH, $-NHR^{11}$, a Li, Na, K, Mg or Ca alkoxide or an oxygen-bonded silyl.

**15.** Poly-3-hydroxyalkanoate carbonyl chlorides of general formula IV according to Claim 11 or poly-3-hydroxyalkanoates of general formula VI according to Claim 12, 13 or 14, **characterized in that** n is 4 to 100.

**16.** Use of the poly-3-hydroxyalkanoate carbonyl chlorides of general formula IV according to Claim 11 or 15 for chemical modification of pharmaceutical active substances.

**17.** Use of the poly-3-hydroxyalkanoates of general formula VI according to any of Claims 12 to 15 in cosmetic compositions, pesticides, adhesives, in textile or leather treatment/care or as plasticizers.

## Revendications

**1.** Procédé de préparation de poly-3-hydroxy-alcanoates de formule générale VI, qui présentent, en une extrémité de chaîne, un motif ester en tant que groupe fonctionnel et, en l'autre extrémité de chaîne, un autre groupe fonctionnel, comprenant les étapes suivantes :

étape 1 : polymérisation avec ouverture de cycle, dans laquelle au moins une β-lactone de formule générale I est transformée avec au moins un acide carboxylique insaturé de formule générale II en présence d'au moins un catalyseur hétérogène en poly-3-hydroxy-alcanoate de formule générale III,

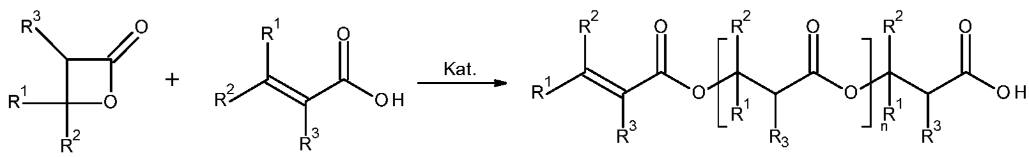


**I** **II** **III**

étape 2 : chloration, dans laquelle le poly-3-hydroxy-alcanoate de formule générale III est transformé avec au moins un agent de chloration Cl, de telle sorte que le chlorure d'acide carboxylique du poly-3-hydroxy-alcanoate de formule générale IV se forme

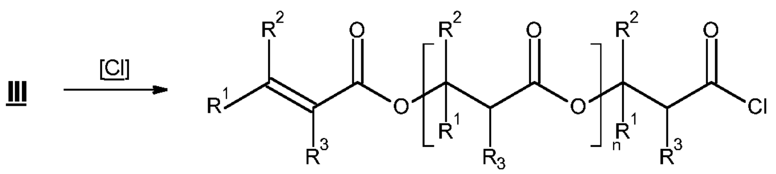


**IV**

et

étape 3 : dérivation, dans lequel le chlorure d'acide carboxylique du poly-3-hydroxy-alcanoate de formule générale IV est transformé avec au moins un composé de formule générale V présentant un groupe fonctionnel X-Y en poly-3-hydroxy-alcanoate de formule générale VI avec dissociation de Y-Cl,

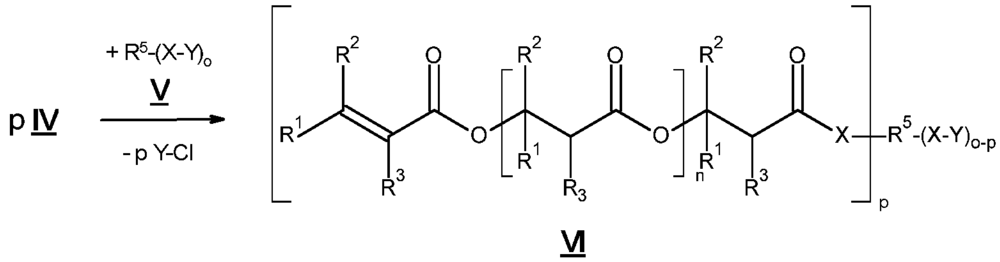

dans lesquelles

$R^1$, $R^2$, $R^3$ signifient, indépendamment les uns des autres, un atome d'hydrogène, un atome d'halogène, un radical hydrocarboné en $C_1$ - $C_{18}$ linéaire, (bi)cyclique ou ramifié, substitué ou non substitué, le cas échéant interrompu par des hétéroatomes choisis parmi O, S ou N, les radicaux $R^1$, $R^2$, $R^3$ pouvant également se trouver sous forme liée par paire en tant qu'élément de structures cycliques,
$R^5$ signifie un radical hydrocarboné linéaire, ramifié ou (bi)cyclique, saturé ou insaturé, monomère ou polymère, substitué ou non substitué, comprenant 1 - 100 atomes de carbone,
X signifie -O- ou $-NR^{11}-$,
où
$R^{11}$ signifie, indépendamment, un atome d'hydrogène ou un radical alkyle linéaire, cyclique ou ramifié, substitué ou non substitué, comprenant 1 à 18 atomes de carbone ou un radical aryle comprenant 6 à 18 atomes de carbone ou un radical de formule générale $-C(=O)-R^{12}$,
où
$R^{12}$ signifie un atome d'hydrogène ou un radical alkyle linéaire, cyclique ou ramifié, substitué ou non substitué, comprenant 1 à 18 atomes de carbone ou un radical aryle comprenant 6 à 18 atomes de carbone ou un radical amine $-NR^8R^9$ primaire, secondaire ou tertiaire,
où
$R^8$ et $R^9$ signifient, indépendamment l'un de l'autre, un atome d'hydrogène ou un radical hydrocarboné comprenant 1 à 6 atomes de carbone et les deux radicaux $R^8$ et $R^9$ peuvent se trouver sous forme liée par paire en tant qu'élément de structures cycliques,
Y signifie un atome d'hydrogène, un radical métallique ou un radical contenant du métal, le métal étant choisi parmi les métaux alcalins, les métaux alcalino-terreux, le silicium, le titane, le zinc, l'étain, le fer, le manganèse ou le cuivre,
n signifie 4 à 1400,
o signifie 1 à 16
et

$$p \leq o.$$

2. Procédé selon la revendication 1, **caractérisé en ce qu'**on utilise, comme β-lactone de formule générale I, une β-lactone de qualité technique présentant une pureté au moins ≥ 90% en poids.

3. Procédé selon la revendication 1 ou 2, **caractérisé en ce qu'**au moins un acide carboxylique insaturé de formule générale II, qui correspond à un isomère de la β-lactone de formule générale I, fonctionne comme initiateur pour la polymérisation avec ouverture de cycle.

4. Procédé selon la revendication 1, 2 ou 3, **caractérisé en ce qu'**on utilise, comme catalyseur, un catalyseur solide, basique, choisi dans le groupe des oxydes, hydroxydes, carbonates et hydrogénocarbonates de métal alcalin ou des fluorures de métal alcalin, le cas échéant sur des matériaux support ou des oxydes ou hydroxydes de métal alcalino-terreux.

5. Procédé selon l'une des revendications 1 à 4, **caractérisé en ce qu'**on utilise, comme agent de chloration Cl, du chlorure de thionyle, du phosgène ou du chlorure de phosphore (V).

6. Procédé selon l'une des revendications 1 à 5, **caractérisé en ce que**, dans un fonctionnement par lots, au moins deux étapes consécutives sont fabriquées sans purification intermédiaire dans la même cuve de réaction ou, dans un procédé semi-continu ou continu, toutes les étapes sont fabriquées dans des réacteurs disposés les uns derrière les

autres.

7. Procédé selon l'une des revendications 1 à 6, **caractérisé en ce que** $R^5$ signifie un radical hydrocarboné linéaire, ramifié ou (bi)cyclique, saturé ou insaturé, monomère ou polymère, substitué ou non substitué, comprenant 3 - 100 atomes de carbone,

8. Procédé selon l'une des revendications 1 à 7, **caractérisé en ce que** le groupe fonctionnel X-Y signifie -OH, $-NHR^{11}$, un alcoolate de Li, Na, K, Mg ou Ca ou un silyle lié par oxygène.

9. Procédé selon l'une des revendications 1 à 8, **caractérisé en ce que en ce que** n signifie 4 à 100.

10. Procédé selon l'une des revendications 1 à 9, **caractérisé en ce que** les étapes 1 à 3 sont réalisées en l'absence de solvant.

11. Chlorures d'acide carboxylique de poly-3-hydroxy-alcanoate de formule générale

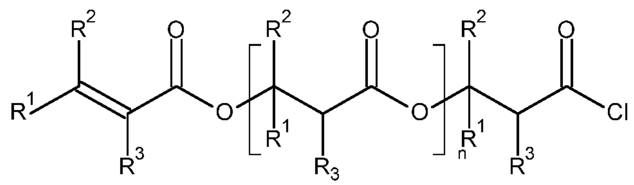

**IV**

dans laquelle

$R^1$, $R^2$, $R^3$ signifient, indépendamment les uns des autres, un atome d'hydrogène, un atome d'halogène, un radical hydrocarboné en $C_1$ - $C_{18}$ linéaire, (bi)cyclique ou ramifié, substitué ou non substitué, le cas échéant interrompu par des hétéroatomes choisis parmi O, S ou N, les radicaux $R^1$, $R^2$, $R^3$ pouvant également se trouver sous forme liée par paire en tant qu'élément de structures cycliques
et
n signifie 4 à 1400.

12. Poly-3-hydroxy-alcanoates de formule générale

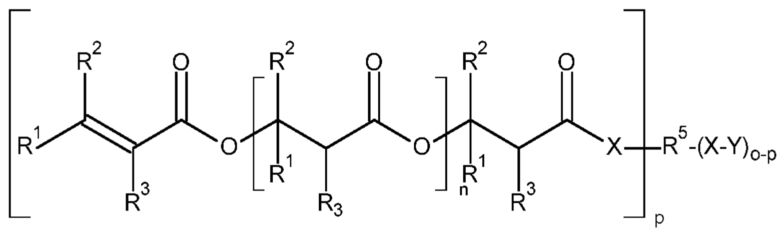

**VI**

dans laquelle

$R^1$, $R^2$, $R^3$ signifient, indépendamment les uns des autres, un atome d'hydrogène, un atome d'halogène, un radical hydrocarboné en $C_1$ - $C_{18}$ linéaire, (bi)cyclique ou ramifié, substitué ou non substitué, le cas échéant interrompu par des hétéroatomes choisis parmi O, S ou N, les radicaux $R^1$, $R^2$, $R^3$ pouvant également se trouver sous forme liée par paire en tant qu'élément de structures cycliques,
$R^5$ signifie un radical hydrocarboné linéaire, ramifié ou (bi)cyclique, saturé ou insaturé, monomère ou polymère, substitué ou non substitué, comprenant 1 - 100 atomes de carbone,
X signifie -O- ou $-NR^{11}-$,
où
$R^{11}$ signifie, indépendamment, un atome d'hydrogène ou un radical alkyle linéaire, cyclique ou ramifié, substitué ou non substitué, comprenant 1 à 18 atomes de carbone ou un radical aryle comprenant 6 à 18 atomes de

carbone ou un radical de formule générale $-C(=O)-R^{12}$,
où
$R^{12}$ représente un atome d'hydrogène ou un radical alkyle linéaire, cyclique ou ramifié, substitué ou non substitué, comprenant 1 à 18 atomes de carbone ou un radical aryle comprenant 6 à 18 atomes de carbone ou un radical amine $-NR^8R^9$ primaire, secondaire ou tertiaire, où
$R^8$ et $R^9$ signifient, indépendamment l'un de l'autre, un radical hydrogène ou un radical hydrocarboné comprenant 1 à 6 atomes de carbone et les deux radicaux $R^8$ et $R^9$ peuvent se trouver sous forme liée par paire en tant qu'élément de structures cycliques,
Y signifie un atome d'hydrogène, un radical métallique ou un radical contenant du métal, le métal étant choisi parmi les métaux alcalins, les métaux alcalino-terreux, le silicium, le titane, le zinc, l'étain, le fer, le manganèse ou le cuivre,
n signifie 4 à 1400,
o signifie 1 à 16
et

$$p < o.$$

**13.** Poly-3-hydroxy-alcanoates de formule générale VI selon la revendication 12, $R^5$ signifiant un radical hydrocarboné linéaire, ramifié ou (bi)cyclique, saturé ou insaturé, monomère ou polymère, substitué ou non substitué, comprenant 3 - 100 atomes de carbone.

**14.** Poly-3-hydroxy-alcanoates de formule générale VI selon la revendication 12 ou 13, **caractérisés en ce que** le groupe fonctionnel X-Y signifie -OH, $-NHR^{11}$, un alcoolate de Li, Na, K, Mg ou Ca ou un silyle lié par oxygène.

**15.** Chlorures d'acide carboxylique de poly-3-hydroxy-alcanoate de formule générale IV selon la revendication 11 ou poly-3-hydroxy-alcanoates de formule générale VI selon la revendication 12, 13 ou 14, **caractérisés en ce que** n signifie 4 à 100.

**16.** Utilisation des chlorures d'acide carboxylique de poly-3-hydroxy-alcanoate de formule générale IV selon la revendication 11 ou 15 pour la modification chimique de principes actifs pharmaceutiques.

**17.** Utilisation des poly-3-hydroxy-alcanoates de formule générale VI selon l'une des revendications 12 à 15 dans des compositions cosmétiques, des pesticides, des adhésifs, dans le traitement/l'entretien des textiles ou du cuir ou comme plastifiant.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010118128 A
- WO 2022143914 A
- US 5223595 A
- DE 4116014 A1


### In der Beschreibung aufgeführte Nicht-Patentliteratur


- **V. SHARMA et al.** *Polymer*, 2021, vol. 212, 123161
- **G. ADAMUS et al.** *Polymers*, 2021, vol. 13, 4365
- **KWIECIEŃ et al.** *PLoS ONE*
- **L. MA et al.** *Macromol. Biosci.*, 2019, vol. 19, 1800432
- **M. A. ABDELWAHAB et al.** *International Journal of Biological Macromolecules*, 2019, vol. 122, 793
- **A. DUDA**. *Journal of Polymer Science: Part A: Polymer Chemistry*, 1992, vol. 30, 21
- **Z. GROBELNY et al.** *Polymer Bulletin*, 2019, vol. 76, 4951
- **M. MICHALAK et al.** *Polymer Degradation and Stability*, 2012, vol. 97, 1861